# FASCICULE DE BREVET EUROPEEN

(11) **EP 0 998 471 B1**
(45) Date de publication et mention de la délivrance du brevet: **14.03.2007**
(21) Numéro de dépôt: 98925703.5
(22) Date de dépôt: 14.05.1998
(51) Int. Cl.: C07D 311/58, C07D 493/04, C07D 311/60, C07D 311/80, C07D 319/20, C07D 335/06, C07D 339/08, A61K 31/35, A61K 31/335, A61K 31/38, A61K 31/385

(54) **COMPOSES HETEROCYCLIQUES, LEUR PROCEDE DE PREPARATION ET LES COMPOSITIONS PHARMACEUTIQUES QUI LES CONTIENNENT**
HETEROZYCLISCHE VERBINDUNGEN, IHR HERSTELLUNGSVERFAHREN UND DIESE ENTHALTENDE PHARMAZEUTISCHE ZUSAMMENSETZUNGEN
SUBSTITUTED HETEROCYCLIC COMPOUNDS, METHOD FOR PREPARING AND COMPOSITIONS CONTAINING SAME

(30) Priorité: 16.05.1997 FR 9706019
(43) Date de publication de la demande: 10.05.2000
(73) Titulaire: Les Laboratoires Servier, 92200 Neuilly sur Seine (FR)
(72) Inventeur: GUILLAUMET, Gérald, F-45650 Saint Jean le Blanc (FR); VIAUD, Marie-Claude, F-45000 Orléans (FR); MAMAI, Ahmed, F-45000 Orléans (FR); CHARTON, Isabelle, F-91330 Yerres (FR); RENARD, Pierre, F-78000 Versailles (FR); BENNEJEAN, Caroline, F-94220 Charenton le Pont (FR); GUARDIOLA, Béatrice, F-92210 Saint Cloud (FR); DAUBOS, Philippe, F-45430 Mardie (FR)
(86) Numéro de dépôt international: PCT/FR1998/000954
(87) Numéro de publication internationale: WO 1998/052935

(56) Documents cités:
- EP-A- 0 300 908
- EP-A- 0 335 375
- EP-A- 0 359 400
- EP-A- 0 389 425
- EP-A- 0 447 285
- EP-A- 0 508 425
- EP-A- 0 527 687
- EP-A- 0 591 057
- EP-A- 0 708 099
- EP-A- 0 721 938
- EP-A- 0 721 947
- EP-A- 0 737 685
- EP-A- 0 745 583
- EP-A- 0 745 584
- EP-A- 0 745 586
- WO-A-94/21608
- WO-A-95/17405
- WO-A-95/29173
- FR-A- 2 203 632
- FR-A- 2 396 011
- FR-A- 2 424 741
- FR-A- 2 583 266
- US-A- 3 509 170
- US-A- 3 663 702
- CHEMICAL ABSTRACTS, vol. 120, no. 21, 23 mai 1994 Columbus, Ohio, US; abstract no. 261501u, XP002077589 & D. SUGDEN: EUR. J. PHARMACOL., vol. 254, no. 3, 1994, pages 271-275,
- CHEMICAL ABSTRACTS, vol. 103, no. 5, 5 août 1985 Columbus, Ohio, US; abstract no. 37451, XP002077590 & A.YA. BUSHKOV ET AL.: KHIM. GETEROTSIKL. SOEDIN., vol. 4, 1985, pages 565-566,
- CHEMICAL ABSTRACTS, vol. 107, no. 23, 7 décembre 1987 Columbus, Ohio, US; abstract no. 217402, XP002077591 & L.I. SHEVCHENKO ET AL.: KHIM. GETEROTSIKL. SOEDIN., vol. 2, 1987, pages 179-181,
- HAHN H -G ET AL: "SYNTHESIS OF 1,4-BENZOXATHIIN DERIVATIVE" JOURNAL OF THE KOREAN CHEMICAL SOCIETY, vol. 38, no. 10, 1994, pages 776-781, XP002053570 cité dans la demande
- CHEMICAL ABSTRACTS, vol. 103, no. 9, 2 septembre 1985 Columbus, Ohio, US; abstract no. 71160, XP002053571 & H. ISHIBASHI ET AL.: CHEM. PHARM. BULL., vol. 33, no. 1, 1985, pages 90-95,
- CHEMICAL ABSTRACTS, vol. 96, no. 9, 1 mars 1982 Columbus, Ohio, US; abstract no. 68760, XP002053572 & Y. TAMURA ET AL.: TETRAHEDRON LETT., vol. 22, no. 38, 1981, pages 3773-3774,
- PATENT ABSTRACTS OF JAPAN vol. 096, no. 001, 31 janvier 1996 & JP 07 242543 A (TAIHO YAKUHIN KOGYO K.K.), 19 septembre 1995
- PATENT ABSTRACTS OF JAPAN vol. 096, no. 001, 31 janvier 1996 & JP 07 242655 A (TAIHO YAKUHIN KOGYO K.K.), 19 septembre 1995
- CHEMICAL ABSTRACTS, vol. 121, no. 21, 21 novembre 1994 Columbus, Ohio, US; abstract no. 255380, XP002077592 & P. DEPREUX ET AL.: J, MED. CHEM., vol. 37, no. 20, 1994, pages 3231-3239,
- CHEMICAL ABSTRACTS, vol. 126, no. 6, 10 février 1997 Columbus, Ohio, US; abstract no. 70291, XP002077593 & D.-H. CAIGNARD: ADV. PINEAL RES., vol. 8, 1994, pages 349-356,
- CHEMICAL ABSTRACTS, vol. 74, no. 5, 1 février 1971 Columbus, Ohio, US; abstract no. 22635, XP002077594 & E. CAMPAIGNE ET AL.: J. MED. CHEM., vol. 13, no. 6, 1970, pages 1205-1208,
- CHEMICAL ABSTRACTS, vol. 81, no. 25, 23 décembre 1974 Columbus, Ohio, US; abstract no. 163634, XP002077595 & R.P. MAICKEL ET AL.: LIFE SCI., vol. 14, no. 9, 1974, pages 1735-1739,
- CHEMICAL ABSTRACTS, vol. 90, no. 19, 7 mai 1979 Columbus, Ohio, US; abstract no. 151901, XP002077596 & E. CAMPAIGNE ET AL.: J. HETEROCYCL. CHEM., vol. 15, no. 8, 1978, pages 1351-1359,
- CHEMICAL ABSTRACTS, vol. 98, no. 23, 6 juin 1983 Columbus, Ohio, US; abstract no. 197920, XP002077597 & E. CAMPAIGNE ET AL.: J. HETEROCYCL. CHEM., vol. 20, no. 1, 1983, pages 55-59,
- CHEMICAL ABSTRACTS, vol. 100, no. 23, 4 juin 1984 Columbus, Ohio, US; abstract no. 191684, XP002077598 & E. CAMPAIGNE ET AL.: J. HETEROCYCL. CHEM., vol. 20, no. 6, 1983, pages 1697-1703,
- CHEMICAL ABSTRACTS, vol. 125, no. 23, 2 décembre 1996 Columbus, Ohio, US; abstract no. 293301y, XP002053574 & H. PICKERING ET AL.: BR. J. PHARMACOL., vol. 119, no. 2, 1996, pages 379-387,

## Description

L'invention concerne de nouveaux dérivés hétérocycliques substitués, leur procédé de préparation et les compositions pharmaceutiques qui les contiennent. Ces nouveaux composés s'avèrent être de puissants ligands des récepteurs mélatoninergiques.

On connaît dans l'art antérieur des dérivés benzoxathiiniques utilisés en tant que fongicides (Hahn H.G. et al., J. Korean Chem. Soc., 1994, 38(10), pp. 776-81) ou en tant qu'inhibiteurs de la lipogénèse chez les mammifères (US 4308276).

On connaît également de nombreux dérivés (dihydro)chromènes ligands 5-HT utiles pour le traitement de l'hypertension, de la dépression ou de l'anxiété (WO 9426703, DE 4135474), ou agonistes des récepteurs dopaminergiques (WO 9608489) utiles dans le traitement de maladies cardiovasculaires.

D'autres dérivés (dihydro)chromènes sont décrits en tant que ligands mélatoninergiques comme par exemple dans les brevets EP 0708099 et EP 0745584 ou dans la demande WO 9529173 qui décrivent des composés substitués sur la partie aromatique de la molécule.

Des dérivés (dihydro)benzodioxiniques sont par ailleurs décrits en tant qu'antioxydants et inhibiteurs de la peroxydation lipidique (EP 624582), ou utiles dans le traitement des maladies du foie (J07242655 et J07242543), ou encore bloquants de récepteurs α-adrénergiques (Dewar G.H. et al., Eur. J. Med. Chem. - Chim. Ther., 1983, 18(3), pp 286-90).

De nombreuses études ont mis en évidence ces dix dernières années, le rôle capital de la mélatonine (5-méthoxy N-acétyl tryptamine) dans le contrôle du rythme circadien et des fonctions endocrines. De surcroît, les récepteurs de la mélatonine ont été caractérisés et localisés.

Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Sécrétions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et sur le traitement de l'obésité (International Journal of Eating Disorders, 1996, 20 (4), pp 443-446).

Des composés permettant d'agir sur le système mélatoninergique sont donc pour le clinicien d'excellents médicaments pour le traitement des pathologies liées au système mélatoninergique et notamment celles mentionnées précédemment.

La présente invention concerne plus particulièrement les composés de formule (I): 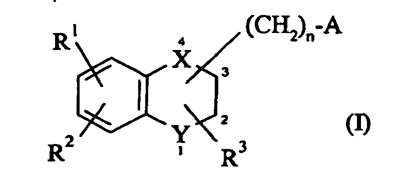 dans laquelle
◆ R¹ représente un groupement OR⁴ dans lequel R⁴ représente un atome d'hydrogène, un groupement alkyle (C₁-C₆) linéaire ou ramifié substitué ou non, alcényle (C₂-C₆) linéaire ou ramifié substitué ou non, alcynyle (C₂-C₆) linéaire ou ramifié substitué ou non, aryle, arylalkyle (C₁-C₆) linéaire ou ramifié, cycloalkyle (C₃-C₈) substitué ou non, ou cycloalkyl (C₃-C₈) alkyle (C₁-C₆) linéaire ou ramifié substitué ou non,
◆ R² représente un atome d'hydrogène,
   ou R¹ et R², situés sur deux carbones adjacents, forment ensemble avec les atomes de carbone qui les portent, un groupement phényle ou phényle substitué,
◆ X représente un groupement CH ou CH₂,
◆ Y représente un atome d'oxygène,
◆ R³ représente un atome d'hydrogène, un groupement aryle, arylalkyle (C₁-C₆) linéaire ou ramifié, ou alkyle (C₁-C₆) linéaire ou ramifié,
◆ n vaut 0, 1, 2, 3, 4 ou 5 lorsque R¹ et R², situés sur deux carbones adjacents, forment ensemble, avec les atomes de carbone qui les portent un groupement phényle ou phényle substitué,
   ou n vaut 1, 2, 3, 4 ou 5 lorsque R² représente un atome d'hydrogène,
◆ A représente
   - un groupement NR⁵R⁶ dans lequel
      R⁶ représente un atome d'hydrogène ou un groupement alkyle (C₁-C₆) linéaire ou ramifié,
      R⁵ représente un groupement 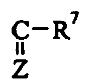 dans lequel Z représente un atome d'oxygène ou un atome de soufre, et R⁷ représente :
      - un atome d'hydrogène,
      - un groupement R⁸ représentant un groupement alkyle (C₁-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, alcényle (C₂-C₆) linéaire ou ramifié substitué ou non, alcynyle (C₂-C₆) linéaire ou ramifié substitué ou non, aryle, ou arylalkyle (C₁-C₆) linéaire ou ramifié,
      - ou un groupement NR⁸R⁹ dans lequel R⁹ représente un atome d'hydrogène ou un groupement alkyle (C₁-C₆) linéaire ou ramifié et R⁸ est tel que défini précédemment,
   - ou un groupement 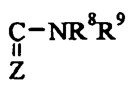
   dans lequel Z, R⁸ et R⁹ sont tels que définis précédemment
étant entendu que :
- le terme "substitué" affecté au terme "phényle" signifie que ce groupement est substitué par un ou plusieurs atomes d'halogène, ou un ou plusieurs groupements, identiques ou différents, choisis parmi, OH, alkoxy (C₁-C₆) linéaire ou ramifié, alkyle (C₁-C₆) linéaire ou ramifié, cyano, nitro, amino, alkylamino, dialkylamino ou trihalogénoalkyle,
- le terme "substitué" affecté aux termes "alkyle", "alcényle" et "alcynyle" signifie que ce groupement est substitué par un ou plusieurs atomes d'halogène, ou un ou plusieurs groupements, identiques ou différents, choisis parmi OH, alkoxy (C₁-C₆) linéaire ou ramifié, amino, alkylamino ou dialkylamino,
- le terme "substitué" affecté aux termes "cycloalkyle" et "cycloalkylalkyle" signifie que la partie cyclique est substituée par un ou plusieurs atomes d'halogène, ou un ou plusieurs groupements, identiques ou différents, choisis parmi alkyle (C₁-C₆) linéaire ou ramifié, alkoxy (C₁-C₆) linéaire ou ramifié, hydroxy, oxo, amino, alkylamino ou dialkylamino,
étant entendu que :
- lorsque le composé de formule (I) est un dérivé chromane (X représente un groupement CH₂, Y représente un atome d'oxygène et R¹, R² et R³ sont tels que définis précédemment), alors le groupement -(CH₂)ₙ-A est différent des groupements suivants :
   - CH₂-NHCORₑ (où Rₑ représente un groupement cycloalkyle) en position 2 du noyau chromane,
   - CH₂-CONHR_{f} (où R_{f} représente un groupement benzyle ou 1-phényl-2-hydroxyéthyle) en position 4 du noyau chromane,
- lorsque A représente un groupement NHCSNHR⁸ et n vaut 2, alors R⁸ ne peut représenter un groupement aryle,
- lorsque X représente un groupement CH₂, R¹ est tel que défini précédemment et R² représente un atome d'hydrogène, alors A ne peut représenter un groupement urée ou thiourée substituée par un noyau phényle (substitué ou non),
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de l'invention sont les composés de formule (I), pous lesquels :
◆ R¹ et R², situés sur deux carbones adjacents, forment ensemble avec les atomes de carbone qui les portent, un groupement phényle, ou phényle substitué,
◆ R³ représente un atome d'hydrogène,
◆ R³ représente un groupement aryle,
◆ A représente un groupement de formule NR⁵R⁶.

Plus spécifiquement, la présente invention concerne les dérivés dihydrobenzochromènes et dihydrochromènes,

Encore plus spécifiquement, la présente invention concerne les composés de formule (I) qui sont :
◆ le N-(9-méthoxy-2,3-dihydro-*1H*-benzo[*f*]chromèn-2-yl)acétamide
◆ le N-[2-(6-méthoxy-3,4-dihydro-*2H*-chroményl)éthyl]acétamide
◆ le N-[(6-méthoxy-*2H*-3-chroményl)méthyl]butanamide.

Les isomères, ainsi que les sels d'addition à un acide ou à une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (II) : 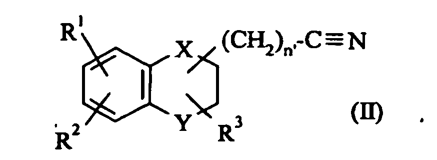 dans laquelle R¹, R², R³, X et Y sont tels que définis précédemment, et n' peut prendre les valeurs de 0 à 4,
que l'on soumet:
◆ à un agent réducteur pour conduire au composé de formule (III) : 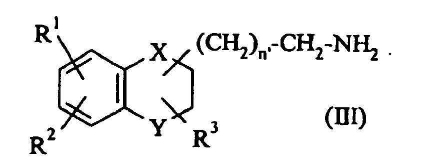 dans laquelle R¹, R², R³, X, Y et n' sont tels que définis précédemment,
   les composés de formule (III) pouvant par ailleurs être obtenus
   - par réduction du composé de formule (IV) : 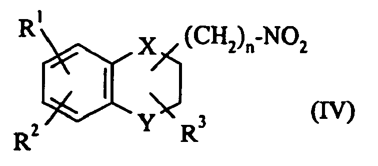 dans laquelle R¹, R², R³, X, Y et n sont tels que définis précédemment,
   - ou à partir du composé de formule (V) : 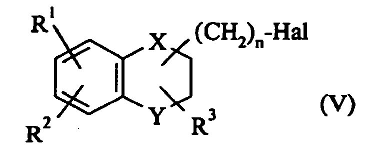 dans laquelle R¹, R², R³, X, Y et n sont tels que définis précédemment et Hal représente un atome d'halogène,
   que l'on substitue par un groupement phtalimide puis que l'on soumet à une hydrazinolyse,
   composé de formule (III) sur lequel on condense :
   - soit un chlorure d'acyle CICOR⁸ ou l'anhydride d'acide (mixte ou symétrique) correspondant pour lesquels R⁸ est tel que défini précédemment,
      pour conduire au composé de formule (I/a), cas particulier des composés de formule (I): 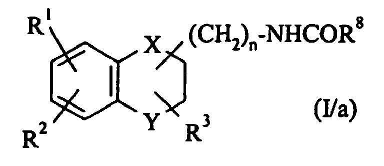 dans laquelle R¹, R², R³, R⁸, X, Y et n sont tels que définis précédemment,
      qui peut être soumis à un agent de thionation comme le réactif de Lawesson pour obtenir le composé de formule (I/b), cas particulier des composés de formule (I) : 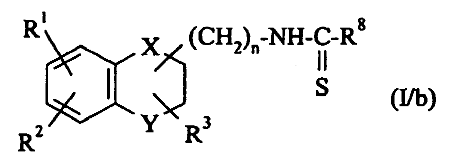 dans laquelle R¹, R², R³, R⁸, X, Y et n sont tels que définis précédemment,
   - soit un composé de formule (VI) :

      Z=C=N-R⁸ (VI)

      dans laquelle Z et R⁸ sont tels que définis précédemment,
      afin d'obtenir le composé de formule (I/c), cas particulier des composés de formule (I) : 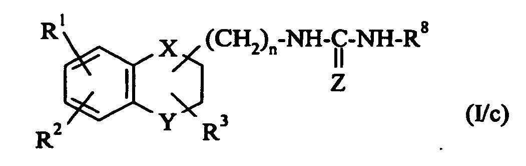 dans laquelle R¹, R², R³, R⁸, X, Y, Z et n sont tels que définis précédemment,
      l'ensemble des composés de formule (I/a), (I/b) et (I/c) formant le composé de formule (I/d), cas particulier des composés de formule (I) : 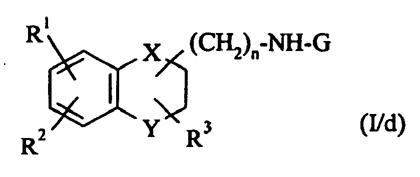 dans laquelle R¹, R², R³, X, Y et n sont tels que définis précédemment, et G représente un groupement COR⁸, CSR⁸ ou CZNHR⁸ dans lesquels Z et R⁸ sont tels que définis précédemment,
      que l'on peut alkyler selon une technique classique d'alkylation grâce à un composé de formule (VII) :

      Alk-W (VII)

      dans laquelle Alk représente un groupement alkyle (C₁-C₆) linéaire ou ramifié et W représente un groupe partant comme un atome d'halogène ou un groupement tosyle,
   ou grâce à un dialkylsulfate,
   pour conduire au composé de formule (I/e), cas particulier des composés de formule (I): 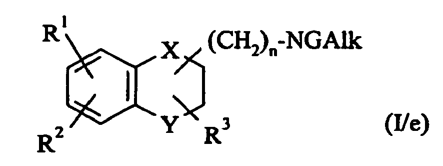 dans laquelle R¹, R², R³, X, Y, G, Alk et n sont tels que définis précédemment,
◆ ou à une hydrolyse en milieu acide ou basique pour conduire au composé de formule (VIII): 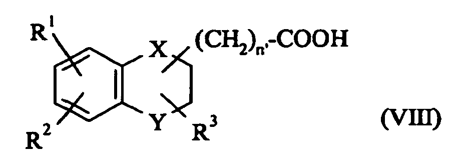
dans laquelle R¹, R², R³, X, Y et n' sont tels que définis précédemment, qui est soumis, après activation sous forme de chlorure d'acide ou en présence d'agent de couplage, à l'action d'une amine H₂NR⁸ où R⁸ est tel que défini précédemment,
pour conduire au composé de formule (I/f), cas particulier des composés de formule (I): 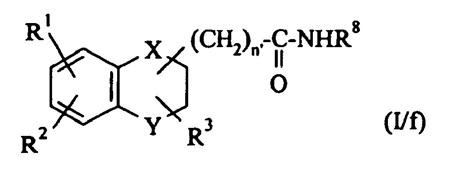 dans laquelle R¹, R², R³, R⁸, X, Y et n' sont tels que définis précédemment,
qui peut être soumis à un agent de thionation comme le réactif de Lawesson pour obtenir le composé (I/g), cas particulier des composés de formule (I) : 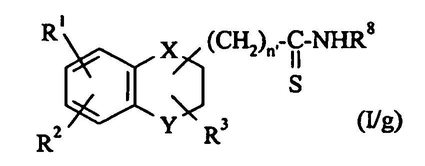 dans laquelle R¹, R², R³, R⁸, X, Y et n' sont tels que définis précédemment,
l'ensemble des composés (I/f) et (I/g) formant le composé de formule (I/h) : 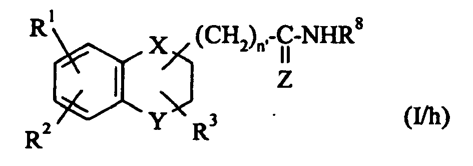 dans laquelle R¹, R², R³, R⁸, X, Y, Z et n' sont tels que définis précédemment,
qui peut être alkylé selon une technique classique d'alkylation pour conduire au composé de formule (I/i) : 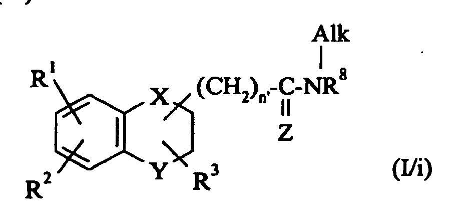 dans laquelle R¹, R², R³, R⁸, X, Y, Z, Alk et n' sont tels que définis précédemment,
les composés de formule (I/a) à (I/i) formant l'ensemble des composés de formule (I) qui peuvent être purifiés selon une technique classique de séparation, que l'on transforme, si on le souhaite en leurs sels d'addition à un acide ou à une base pharmaceutiquement acceptable, et dont on sépare éventuellement les isomères selon une technique classique de séparation.

Les composés de formule (II) sont obtenus entre autres :
- à partir des composés de formule (IX) : 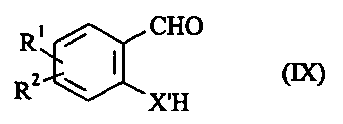 dans laquelle R¹ et R² sont tels que définis précédemment et X' représente un atome de soufre ou un atome d'oxygène,
   que l'on condense sur de l'acrylonitrile, pour conduire au composé de formule (II/a), cas particulier des composés de formule (II) : 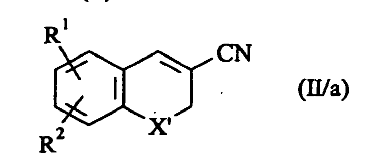 dans laquelle R¹, R² et X' sont tels que définis précédemment,
   que l'on soumet à une réduction pour obtenir le composé de formule (II/b), cas particulier des composés de formule (II) : 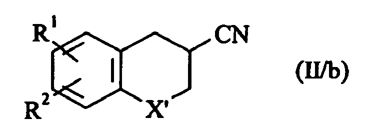 dans laquelle R¹, R² et X' sont tels que définis précédemment,
- à partir des composés de formule (X) : 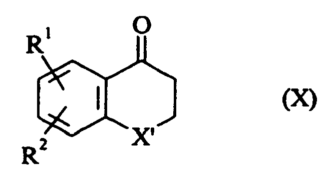 dans laquelle R¹, R² et X' sont tels que définis précédemment,
   que l'on soumet à une réaction de Wittig puis à une réduction catalytique pour obtenir le composé de formule (II/c), cas particulier des composés de formule (II) : 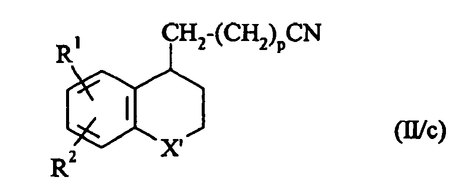 dans laquelle R¹, R² et X' sont tels que définis précédemment et p vaut 0, 1, 2, 3, ou 4,
- à partir des composés de formule (XI) : 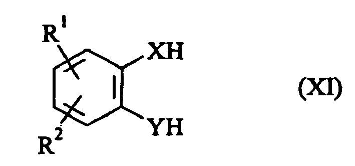 dans laquelle R¹, R², X et Y sont tels que définis précédemment,
   sur lesquels on condense :
   - le chloroacrylonitrile pour obtenir le composé de formule (II/d), cas particulier des composés de formule (II) : 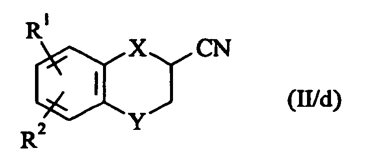 dans laquelle R¹, R² X et Y sont tels que définis précédemment,
      qui peut être dibromé puis traité à l'iodure de sodium pour conduire au composé de formule (II/e), cas particulier des composés de formule (II) : 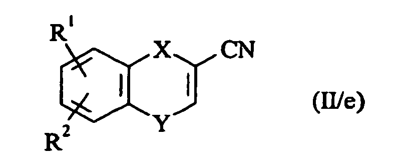 dans laquelle R¹, R², X et Y sont tels que définis précédemment,
   - ou le 2,3-dibromopropionate d'éthyle pour conduire au composé de formule (XII) : 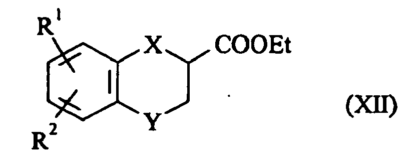 dans laquelle R¹, R², X et Y sont tels que définis précédemment,
      composé (XII) que l'on soumet à une lithiation puis à la condensation de l'électrophile désiré afin de conduire au composé de formule (XV) : 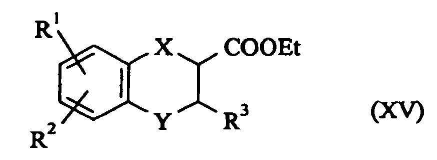 dans laquelle R¹, R², R³, X et Y sont tels que définis précédemment,
      qui peut être successivement réduit en alcool correspondant, oxydé en aldéhyde, et soumis à une réaction de Wittig pour conduire au composé de formule (XVI) : 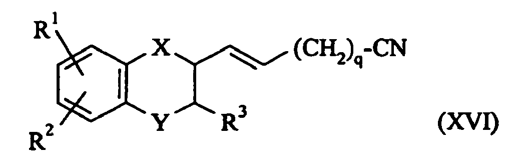 dans laquelle R¹, R², R³, X et Y sont tels que définis précédemment et q vaut 0, 1, 2 ou 3,
      qui est réduit catalytiquement pour conduire au composé de formule (II/f), cas particulier des composés de formule (II) : 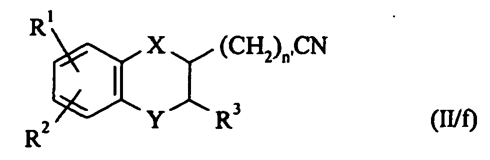 dans laquelle R¹, R², R³, X, Y et n' sont tels que définis précédemment.

Les composés de l'invention et les compositions pharmaceutiques les contenant s'avèrent être utiles pour le traitement des troubles du système mélatoninergique.

L'étude pharmacologique des dérivés de l'invention a en effet montré qu'ils n'étaient pas toxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention possèdent des propriétés d'inhibiteurs de l'ovulation, d'immunomodulateurs et qu'ils sont susceptibles d'être utilisés dans le traitement des cancers.
Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant les produits de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention, mais ne la limitent en aucune façon.

### PREPARATION 2 : 9-Méthoxy-3H-benzo[f]chromène-2-carbonitrile

### Stade A : 2,7-Diméthoxy-naphtalène

La méthylation du 2,7 dihydroxynaphtalène (10 g : 62,43 mmol) s'effectue au sein de l'acétone (100 ml) en présence de diméthylsulfate (12,06 ml : 127 mmol : 2,03 éq.) et de carbonate de potassium sec (42,3 g : 306 mmol : 4,9 éq.). La température du milieu réactionnel est de 56°C pendant 6 heures puis de 40°C durant 12 heures. L'hydrolyse (7,4 ml d'eau) nécessite 2 heures d'agitation à température ambiante. Après filtration des sels sur célite et concentration du filtrat résiduel, l'extraction au dichlorométhane conduit à une phase organique qui, évaporée, prend l'aspect d'un solide beige. Ce dernier est décoloré par le charbon actif puis recristallisé dans un mélange EP/CH₂Cl₂.
Point de fusion: 138°C

### Stade B : 2,7-Diméthoxy-1-naphtaldéhyde

A une solution du dérivé obtenu au Stade A (5 g : 26,3 mmol) dans le dichlorométhane anhydre (50 ml) sont injectés successivement le tétrachlorure de titane (4,09 ml : 37,5 mmol : 1,4 éq.) et l'αα-dichlorométhylméthyléther (3,6 ml : 37,5 mmol : 1,5 éq.) préalablement dissous dans 14 ml de dichlorométhane. Ces opérations s'effectuant à 0°C, la température est graduellement ramenée à 25°C puis maintenue ainsi durant 5 heures. Le mélange réactionnel est alors lentement versé sur de la glace, une solution d'acide chlorhydrique 3N (103 ml) étant ensuite ajoutée avec précautions. Après hydrolyse du complexe, le produit est extrait au dichlorométhane, lavé à l'eau puis à l'aide d'une solution saturée d'hydrogénocarbonate de sodium. Le résidu sec recueilli après concentration est lavé à l'éther diéthylique, permettant ainsi d'isoler l'aldéhyde pur.
Point de fusion: 94°C

### Stade C : 2-Hydroxy-7-méthoxy-1-naphtaldéhyde

Le produit diméthoxylé obtenu au Stade B (2 g : 9,25 mmol) étant en solution dans le dichlorométhane anhydre, le complexe BBr₃Me₂S à 97% (2,98 g : 9,25 mmol : 1 éq.) est introduit à température ambiante. L'hydrolyse est effectuée après 35 minutes d'agitation à l'aide d'une solution saturée d'hydrogénocarbonate de sodium (pH = 8). Le produit est extrait au dichlorométhane puis purifié sur colonne de silice AcOEt/EP (3/7).
Point de fusion: 126°C

### Stade D : 9-Méthoxy-3H-benzo[f]chromène-2-carbonitrile

Sous atmosphère anhydre, l'aldéhyde obtenu au Stade C (2 g : 9,9 mmol) est partiellement solubilisé dans l'acrylonitrile (6,5 ml : 49,4 mmol : 10 éq.). La solution devient limpide après l'ajout de 1,4-diazabicyclo-[2-2-2]-octane (227 mg : 2,47 mmol : 0,25 éq.). Cette dernière se colore en rouge lors du reflux d'une durée de 18 heures. Le milieu réactionnel est alors dilué à l'acétate d'éthyle, lavé successivement avec une solution de soude 1N (20 ml) puis une solution d'acide chlorhydrique 1N (20 ml). Les phases organiques concentrées sont purifiées sur colonne de silice flash éluée par un mélange AcOEt / EP (0,1/1).
Point de fusion : 134°C

### PREPARATION 11 : 6-Méthoxy-2H-3-chromène carbonitrile

Une solution de 0,61 g (4 mmol) de 2-hydroxy-5-méthoxy benzaldéhyde et 0,112 g (1 mmol) de 1,4-diazabicyclo[2.2.2]octane dans 18 ml d'acrylonitrile est portée à reflux pendant 24 heures sous argon. Après refroidissement, le milieu est dilué avec du chloroforme puis lavé avec une solution d'hydrogénocarbonate de sodium saturée. La phase organique est ensuite acidifiée avec une solution d'acide chlorhydrique (1N) puis la phase aqueuse est extraite au chloroforme. Les phases organiques, séchées sur sulfate de magnésium puis filtrées, sont concentrées sous pression réduite. Le nitrile du titre est obtenu pur sous forme d'un solide jaune après passage sur colonne de silice (éluant : AcOEt/EP : 25/75).
Point de fusion : 70-71°C

### PREPARATION 14 : 9-Méthoxy-2,3-dihydro-1H-benzo[f]chromèn-1-one

### Stade A : 3(7-Méthoxy-1,4-dihydro-2-naphtalényl)oxy]propanenitrile

On procède comme dans le stade A de l'Exemple 12 à partir du 7-méthoxy-1,4-dihydro-2-naphtalénol.

### Stade B : 9-Méthoxy-2,3-dihydro-1H-benzo[f]chromèn-1-one

On procède comme dans les stades B et C de l'Exemple 12.

### PREPARATION 15 : 9-Méthoxy-2,3-dibydro-1H-benzo[f]chromène-1-carbonitrile

En milieu anhydre, la cétone obtenue dans la Préparation 14 (500 mg, 2,19 mmol), le diéthylcyanophosphonate (2 éq; 4,38 mmol., 715 µl) ainsi que le cyanure de lithium à 0,5 M en solution dans le DMF (3 éq; 6,57 mmol., 13,15 ml) sont mélangés dans 20 ml de THF. Après 30 minutes d'agitation, le milieu réactionnel est hydrolysé, puis extrait à l'AcOEt. En parallèle, une solution de iodure de samarium est préparée. Le samarium (4,5 éq; 9,86 mmol.; 1,48 g) est mis en suspension dans 10 ml de THF, puis goutte à goutte le diiodoéthane (3 éq; 6,57 mmol.; 1,85 g) dilué dans 10 ml de THF, est additionné. Lorsque la solution d'iodure de samarium est devenue bleue, le complexe formé précédemment est dissous dans 5 ml de THF et 0,21 ml de *tert*-butanol, puis additionné. La solution est laissée sous agitation pendant 12 heures à température ambiante. Le milieu réactionnel est hydrolysé avec une solution d'HCl à 10 %. Après extraction à l'AcOEt, la phase organique est lavée avec une solution de Na₂S₂O₃ à 10 %, puis deux fois avec une solution saturée de NaHCO₃. Le résidu obtenu est purifié par chromatographie "flash" sur gel de silice (éluant : EP/AcOEt (8/2)). Le produit du titre est obtenu sous la forme d'une huile.

### PREPARATION 16 : 2-(9-Méthoxy-2,3-dibydro-1H-benzo[f]chromèn-1-yl) acétonitrile

### Stade A : 2-(9-Méthoxy-2,3-dihydro-1H-benzo[f]chromèn-1-yliden)acétonitrile

En milieu anhydre, le diéthylecyanométhylphosphonate (3 éq; 17,1 mmol.; 2,76 ml) est additionné lentement à une suspension d'hydrure de sodium à 50 % (3 éq; 17,1 mmol.; 820 mg) dans 50 ml de THF, à 0°C. Le milieu réactionnel est agité durant 10 minutes à 0°C puis refroidi à -78°C. La cétone obtenue dans Préparation 14 (1,3 g; 5,7 mmol.), en solution dans 15 ml de THF est ajoutée. La température est alors ramenée lentement à 20-25°C durant 2 heures 30. Après élimination du solvant, le composé est extrait à l'AcOEt. La phase organique est abondamment lavée avec une solution saturée de NaCl, puis concentrée sous pression réduite. Le produit désiré est obtenu sous forme d'un solide.
Point de fusion: 61-63°C

### Stade B : 2-(9-Méthoxy-2,3-dihydro-1H-benzo[f]chromèn-1-yl)acétonitrile

Le composé insaturé obtenu au stade A (1,775 éq; 7,07 mmol.) solubilisé dans 50 ml d'éthanol et quelques gouttes de THF, est introduit dans le réacteur de Parr. Le palladium sur charbon à 10 % (266 mg; 15 % en masse) est alors additionné. On laisse sous une presion d'hydrogène de 45 Psi, sous agitation, pendant 18 heures. Après filtration sur célite, le solvant est éliminé sous pression réduite. Le résidu est purifié par chromatographie "flash" sur gel de silice (éluant : EP/AcOEt (9/1)). Le produit désiré est obtenu sous la forme d'un solide.
Point de fusion : 122-123°C

### EXEMPLE 4 : N-[(9-Méthoxy-2,3-dihydro-1H-benzo[f]chromèn-2-yl)méthyl] acétamide

### Stade A : 9-Méthoxy-2,3-dihydro-1H-benzo[f]chromène-2-carbonitrile

200 mg (0,93 mmol) du produit obtenu dans la Préparation 2 sont introduits dans l'appareil de Parr de l'hydrogénateur avec 15 ml d'éthanol et 20 mg de palladium sur charbon à 10%. Le mélange est agité sous atmosphère d'hydrogène pendant 24 h. On filtre sur célite puis on évapore l'éthanol. Le produit du titre est purifié sur colonne de silice (éluant : EP/AcOEt, 1/1).

### Stade B : N-[(9-Méthoxy-2,3-dihydro-1H-benzo[f]chromèn-2-yl)méthyl]acétarnide

175 mg du produit obtenu au Stade A sont dissout dans 10 ml d'éther distillé. A cette solution, on ajoute 77 mg de LiAlH₄. On laisse la réaction 24 h à température ambiante. On hydrolyse ensuite le mélange avec 0,08 ml d'eau, 0,08 ml de NaOH 15 % et enfin 0,25 ml d'eau. On filtre sur célite, on lave à l'acétate d'éthyle que l'on évapore. Le produit du titre est purifié sur colonne de silice (éluant CH₂Cl₂/MeOH : 90/10).
Dans un ballon, le produit précédemment obtenu est dissous dans 5 ml de CH₂Cl₂. On additionne, à 0°C dans la glace, 0,2 ml de pyridine et 0,2 ml d'anhydride acétique. On laisse agir pendant 2 h. Le produit est ensuite hydrolysé puis extrait au dichlorométhane. On concentre la phase organique séchée sur MgSO₄, puis on évapore avec du toluène le reste de pyridine. Le produit du titre est purifié sur colonne de silice (éluant : CH₂Cl₂/MeOH) puis recristallisé dans l'éthanol et le cyclohexane.
Point de fusion : 138°C

| Microanalyse Elémentaire : | | | |
|---|---|---|---|
| | C | H | N |
| % Calculé | 71,56 | 6,71 | 4,91 |
| % Trouvé | 71,50 | 6,79 | 4,86 |

### EXEMPLE 5 : N-(9-Méthoxy-2,3-dibydro-1H-benzo[f]chromèn-2-yl)acétamide

### Stade A : Acide 9-méthoxy-3H-benzo[f]chromène-2-carboxylique

Un mélange du dérivé cyané obtenu dans la Préparation 2 (1 g : 4,21 mmol) et d'une solution de soude à 10% (34 ml : 84,3 mmol : 20 éq.) est porté à reflux durant 5 heures. Le milieu réactionnel refroidi est extrait à pH basique à l'acétate d'éthyle. La précipitation de l'acide est effectuée à froid par acidification de la phase aqueuse résiduelle à l'aide d'acide chlorhydrique 2N puis 3N. Le solide recueilli par filtration est séché sous vide en présence de pentoxyde de phosphore.
Point de fusion : 226°C

### Stade B : Acide 9-méthoxy-2,3-dihydro-1H-benzo[f]chromène-2-carboxylique

L'acide insaturé obtenu au Stade A (750 mg : 2,9 mmol) est solubilisé dans un mélange éthanol/diméthylformamide (20 ml : 3 ml) dans le réacteur de l'appareil de Paar. Après l'ajout du catalyseur, le palladium sur charbon à 10% (75 mg : 10 % en masse), le tout est agité à température ambiante sous une pression de 45 psi durant 4 heures. Les solvants sont éliminés, puis l'huile résiduelle est précipitée et lavée à l'éther diéthylique.
Point de fusion : 165°C

### Stade C : N-(9-Méthoxy-2,3-dihydro-1H-benzo[f]chromèn-2-yl)acétamide

L'acide obtenu au Stade B (50 mg : 0,19 mmol) étant en solution dans l'acétone (2 ml) sous atmosphère inerte, la triéthylamine distillée (0,07 ml : 0,52 mmol : 2,7 éq.) ainsi que le chloroformiate d'éthyle distillé (0,06 ml : 0,68 mmol : 3,5 éq.) sont introduits successivement et lentement à 0°C. Après 30 minutes d'agitation l'azoture de sodium (57 mg : 0,87 mmol : 4,5 éq.) en solution dans 1 ml d'eau est ajouté. Un laps de temps similaire s'étant écoulé, le milieu réactionnel est extrait au dichlorométhane. La phase organique résultante est concentrée, reprise dans 1 ml de toluène et portée à reflux pendant 30 minutes. L'acide acétique glacial (1 ml) est alors introduit à chaud puis le chauffage est prolongé pendant 1 heure 30 minutes. A température ambiante, les produits sont extraits à l'acétate d'éthyle puis purifiés sur colonne de silice flash CHCl₃ / AcOEt 7/3. Le composé du titre est lavé à l'éther diéthylique.
Point de fusion : 186°C

| Microanalyse Elémentaire : | | | |
|---|---|---|---|
| | C | H | N |
| % Calculé | 70,83 | 6,32 | 5,16 |
| % Trouvé | 70,75 | 6,29 | 4,95 |

### EXEMPLE 6 : N-[(9-Méthoxy-2,3-dihydro-1H-benzo[f]chromèn-2-yl)méthyl]-2-cyclopropylacétamide

Le composé du titre est obtenu par le même procédé que pour l'Exemple 4 en remplaçant dans la dernière étape l'anhydride acétique par l'anhydride cyclopropane carboxylique.

### EXEMPLE 7 : N-[(9-Méthox-2,3-dihydro-1H-benzo[f]chromèn-2-yl)méthyl]-1-cyclohexylcarboxamide

L'exemple du titre est obtenu comme dans l'Exemple 5, en utilisant l'acide cyclohexane carboxylique en lieu et place de l'acide acétique.

### EXEMPLE 8 : N-Méthyl-9-méthoxy-2,3-dihydro-1H-benzo[f]chromène-2-carboxamide

La méthylamine, condensée sur l'acide obtenu dans le Stade B de l'Exemple 5, permet d'obtenir le composé du titre.

### EXEMPLE 10 : N-[2-(3,4-Dibydro-2H-4-chroményl)éthyl]acétamide

### Stade A : 2-(3,4-Dihydro-2H-4-chroményliden)acétonitrile

Le cyanométhylphosphonate de diéthyle (1,15 éq.) est additionné lentement à une suspension d'hydrure de sodium (1,15 éq.) dans le tétrahydrofurane anhydre à 0°C. Le milieu réactionnel est agité durant 10 minutes à 0°C puis refroidi à -78°C. La 4-chromanone en solution dans le tétrahydrofurane est ajoutée. La température est alors ramenée lentement à 20-25°C durant 2 heures 30. Après élimination du solvant les composés sont extraits à l'acétate d'éthyle. La phase organique est abondamment lavée avec une solution saturée de chlorure de sodium. Cette dernière concentrée est purifiée sur colonne de silice (AcOEt / EP 3/7) (Mélange Z/E).

### Stade B : 2-(3,4-Dihydro-2H-4-chroményl)acétonitrile

Le composé insaturé obtenu au Stade A (mélange Z + E) solubilisé dans l'éthanol (20 ml) est introduit dans le réacteur de l'appareil de Paar. Le palladium sur charbon à 10 % (en masse) est alors additionné. La double liaison est hydrogénée sous une pression de 45 psi durant 12 heures.

Le catalyseur est éliminé par filtration puis le solvant est évaporé. L'huile résiduelle est purifiée sur colonne de silice éluée par un mélange AcOEt / EP 3/7.
Huile incolore.

### Stade C : N-[2-(3,4-Dihydro-2H-4-chroményl)éthyl]acétamide

Le dérivé cyané saturé obtenu au Stade B est solubilisé dans le réacteur de l'appareil de Paar à l'aide d'anhydride acétique. L'acétate de sodium (1,5 éq.) ainsi que le nickel de Raney (6 mg pour 50 mg de produit) sont alors introduits dans le milieu réactionnel. Le tout est chauffé à 50°C sous une pression d'hydrogène de 40 psi durant 12 heures. Le solvant étant éliminé après retour à des conditions normales de température et de pression, l'extraction s'effectue au moyen d'acétate d'éthyle. Une purification par flash chromatographie (CHCl₃ / AcOEt 7/3) de la phase organique concentrée aboutit à l'amide recherché.
Gomme incolore.

### EXEMPLE 11 : N-[2-(3,4-Dihydro-2H-4-chroményl)éthyl]éthanethioacétamide

Le composé obtenu dans l'Exemple 10, soumis au réactif de Lawesson permet d'obtenir le composé du titre.

### EXEMPLE 12 : N-[2-(6-Méthoxy-3,4-dibydro-2H-4-chroményl)éthyl]acétamide

### Stade A : 2-(4-Méthoxyphénoxy)cyanoéthane

Un mélange du 4-méthoxyphénol (10 g : 80,5 mmol) et de triton B (2,29 ml : 14,5 mmol : 0,18 éq.) dans l'acrylonitrile (50 ml) est porté à reflux durant 48 heures. Le solvant étant partiellement éliminé, les produits sont extraits à l'acétate d'éthyle, lavés à l'eau puis avec une solution d'acide chlorhydrique 6N. L'huile marron résiduelle est purifiée sur une colonne de silice flash éluée par un gradient AcOEt / EP.
Point de fusion: 58°C (solide jaune pâle)

### Stade B : Acide 3-(4-méthoxyphénoxy)propanoïque

L'hydrolyse du dérivé cyané obtenu au Stade A (3 g : 16,93 mmol) s'effectue à l'aide d'acide chlorhydrique concentré à 37% (8,3 ml : 84,3 mmol : 5 éq.) à reflux. Après dissolution du produit de départ le composé désiré précipite au bout de 2 heures de chauffage. Le milieu réactionnel étant refroidi, le solide est filtré, lavé avec un mélange glace-eau, introduit dans une solution de 10 ml d'eau contenant 1 g d'hydrogénocarbonate de sodium. Cette dernière est agitée vivement durant 1 heure puis filtrée. Le filtrat résultant conduit à l'acide pur après acidification à froid (acide chlorhydrique 3N) et filtration.
Point de fusion : 104°C (solide blanc)

### Stade C : 6-Méthoxy-4-chromanone

La cyclisation est effectuée sur le chlorure d'acide généré à partir de l'acide. Le chauffage à reflux de toluène (20 ml) de l'acide obtenu au Stade B (500 mg : 2,55 mmol) durant 3 heures en présence de chlorure de thionyle (0,56 ml : 7,65 mmol : 3 éq.) permet d'accéder aisément à ce dernier. Le solvant ainsi que l'excès de réactif sont éliminés par concentration sous pression réduite de la solution jaune obtenue. Le résidu sec est alors repris dans 20 ml de dichlorométhane anhydre puis le chlorure d'aluminium (465,9 mg : 3,49 mmol : 1,5 éq.) est introduit précautionneusement. Le mélange réactionnel est hydrolysé à froid après 1 heure d'agitation à température ambiante. Le produit est extrait, lavé à l'eau puis purifié par une colonne de silice (AcOEt / EP 3/7).
Point de fusion : 43°C (solide jaune pâle)

### Stade D : 2-(6-Méthoxy-3,4-dihydro-2H-4-chroményliden)acétonitrile

On procède comme dans le Stade A de l'Exemple 10 à partir du composé obtenu au Stade C.

### Stade E : 2-(6-Méthoxy-3,4-dihydro-2H-4-chroményl)acétonitrile

On procède comme dans le Stade B de l'Exemple 10 à partir du composé obtenu au Stade D.
Huile incolore.

### Stade F : N-[2-(6-Méthoxy-3,4-dihydro-2H-4-chroményl)éthyl]acétamide

On procède comme dans le Stade C de l'Exemple 10 à partir du composé obtenu au Stade E.
Point de fusion : 138°C

| Microanalyse Elémentaire : | | | |
|---|---|---|---|
| | C | H | N |
| % Calculé | 67,45 | 7,68 | 5,62 |
| % Trouvé | 66,90 | 7,74 | 5,49 |

### EXEMPLE 13 : N-[2-(6-Méthoxy-3,4-dihydro-2H-4-chroményl)éthyt]butanamide

Le dérivé cyané (500 mg ; 2,46 mmol) obtenu au Stade E de l'Exemple 12 est solubilisé dans le réacteur de Paar avec du méthanol (50 ml). Le nickel de Raney (6 mg pour 50 mg de produit) est alors ajouté. Le mélange est chauffé à 50°C sous une pression d'hydrogène de 40 psi pendant 16 heures. Le nickel est ensuite filtré sur célite, et le méthanol évaporé. Le mélange brut est dissous dans du dichlorométhane (10 ml) anhydre, puis la solution est refroidie à 0°C. Le chlorure de butyryle (1,4 éq. ; 3,36 mmol ; 348 µl), puis la triéthylamine (3 éq. ; 7,2 mmol ; 1,013 ml) sont ajoutés au milieu. Au bout d'une heure d'agitation, le mélange réactionnel est acidifié avec une solution d'acide chlorhydrique (1N). Après extraction au dichlorométhane, la phase organique est lavée avec une solution saturée d'hydrogénocarbonate de sodium, séchée sur sulfate de magnésium, puis concentrée sous vide. Le résidu obtenu est purifié par chromatographie flash avec comme éluant : EP /AcOEt (6/4) puis (1/1) puis (4/6).
Le produit est obtenu sous forme de cristaux blancs après lavage à l'éther et au pentane.
Point de fusion : 63-64°C

| Microanalyse Elémentaire : | | | |
|---|---|---|---|
| | C | H | N |
| % Calculé | 69,29 | 8,36 | 5,05 |
| % Trouvé | 69,30 | 8,31 | 5,05 |

### EXEMPLE 15 : N-[2-(6-Méthoxy-3,4-dihydro-2H-4-chroményl)éthyl]-2-phénylacétamide

On procède comme dans les Stades A, B, C, D et E de l'Exemple 12, puis on réalise une réduction du nitrile dans les conditions du Stade C de l'Exemple 1, puis la condensation du chlorure de phénylacétyle permet d'obtenir le composé du titre.

### EXEMPLE 36 : N-[(6-Méthoxy-3,4-dibydro-2H-3-cbroményl)méthyl]acétamide

0,935 g (5 mmol) du nitrile obtenu dans la Préparation 11 et 0,623 g (7,5 mmol) d'acétate de sodium sont ajoutés à une suspension de 0,44 g de nickel de Raney dans 20 ml d'anhydride acétique puis le mélange est soumis à une pression de 50 psi d'hydrogène à une température de 50°C pendant 24 heures. Après refroidissement, les sels sont filtrés puis le solvant est évaporé sous vide. Le résidu obtenu est repris dans de l'acétate d'éthyle puis la phase organique est lavée avec une solution saturée d'hydrogénocarbonate de sodium. Après séchage sur sulfate de magnésium et filtration, le filtrat est concentré sous vide. Une chromatographie sur colonne de silice (éluant : AcOEt) permet de conduire à l'amide du titre sous forme d'un solide blanc.
Point de fusion : 110°C

| Microanalyse Elémentaire : | | | |
|---|---|---|---|
| | C | H | N |
| % Calculé | 66,36 | 7,28 | 5,95 |
| % Trouvé | 66,54 | 7,40 | 5,90 |

### EXEMPLE 37 : N-[(6-Méthoxy-3,4-dihydro-2H-3-chroményl)méthyl]-1-cyclobutane-carboxamide

On procède comme dans le Stade B de l'Exemple 4 à partir du composé obtenu dans la Préparation 11, puis l'anhydride cyclobutane carboxylique est condensé pour conduire au composé du titre.

### EXEMPLE 38 : N-[2-(6-Méthoxy-3,4-dihydro-2H-3-chroményl)éthyl]acétamide

### Stade A : (6-Méthoxy-3,4-dihydro-2H-3-chroményl)méthanol

2 g (10,5 mmol) de l'alcool obtenu dans la Préparation 12 sont dissous dans 60 ml d'éthanol puis 0,616 g (10,5 mmol) de nickel de Raney préalablement lavé à l'éthanol sont ajoutés à cette solution. Le milieu réactionnel est soumis à une pression d'hydrogène de 50 psi pendant 22 heures. Le catalyseur résiduel est filtré puis le solvant est évaporé sous vide. Une chromatographie sur colonne de silice (éluant : AcOEt/EP : 50/50) permet d'aboutir à l'alcool du titre pur sous forme d'un solide blanc.
Point de fusion : 56-57°C

### Stade B : [(6-Méthoxy-3,4-dihydro-2H-3-chroményl)méthyl]4-méthyl-1-benzènesulfonate

1,83 g (9,44 mmol) de l'alcool obtenu au Stade A sont dissous dans 40 ml de dichlorométhane anhydre puis 3,15 g (16,5 mmol) de chlorure de paratoluène sulfonyle et 4,6 ml (33 mmol) de triéthylamine sont successivement additionnés au milieu. Après 21 heures d'agitation à température ambiante et sous argon, le solvant est évaporé sous pression réduite. Le tosylate du titre est obtenu pur sous forme d'une huile claire après passage sur colonne de silice (éluant : AcOEt/EP : 15/85 puis 30/70).

### Stade C : 2-(6-Méthoxy-3,4-dihydro-2H-3-chroményl)acétonitrile

1,6 g (4,56 mmol) du tosylate obtenu au Stade B est dissous dans 25 ml de N,N-diméthylformamide anhydre puis 0,724 g (11,5 mmol) de cyanure de potassium sont ajoutés à la solution. Après 4 heures de reflux sous argon, le solvant est évaporé sous vide puis le résidu est repris avec une solution saturée d'hydrogénocarbonate de sodium. La phase aqueuse est ensuite extraite au dichlorométhane. Les phases organiques, séchées sur sulfate de magnésium puis filtrées, sont concentrées sous pression réduite. Le nitrile du titre est obtenu pur sous forme d'une huile qui cristallise lentement après une chromatographie sur gel de silice (éluant : AcOEt/EP : 25/75).
Point de fusion : 54-55°C

### Stade D : N-[2-(6-Méthoxy-3,4-dihydro-2H-3-chroményl)éthyl]acétamide

0,812 g (4 mmol) du nitrile obtenu au Stade C sont ajoutés à une suspension de 0,35 g (6 mmol) de nickel de Raney et 0,5 g (6 mmol) d'acétate de sodium dans 40 ml d'anhydride acétique. Le milieu réactionnel est ensuite soumis à une pression d'hydrogène de 50 psi à une température de 50°C pendant 5 heures. Après refroidissement du milieu, les sels sont filtrés et le filtrat est concentré sous pression réduite. Le résidu obtenu est ensuite repris avec de l'acétate d'éthyle puis la phase organique est lavée avec une solution d'hydrogénocarbonate de sodium saturée. Après séchage sur sulfate de magnésium et filtration, le solvant est évaporé sous vide. Une chromatographie sur colonne de silice (éluant : AcOEt) permet d'aboutir à l'amide du titre sous forme d'un solide blanc.
Point de fusion : 114°C

| Microanalyse Elémentaire : | | | |
|---|---|---|---|
| | C | H | N |
| % Calculé | 67,45 | 7,68 | 5,62 |
| % Trouvé | 67,72 | 7,71 | 5,62 |

### EXEMPLE 39 : N-Méthyt-N-phényt-2-(6-méthoxy-3,4-dihydro-2H-3-chmményl)

### acétamide

Le composé du titre est obtenu après hydrolyse du nitrile obtenu au Stade C de l'Exemple 38, transformation en chlorure d'acide correspondant et condensation de la N-Méthyl-N-Phénylamine.

### EXEMPLE 46 : N-[(6-Méthoxy-3,4-dibydro-2H-4-chroményl)méthyl]acétamide

### Stade A : 6-Méthoxy-4-chromanecarbonitrile

En milieu anhydre, le composé obtenu au stade C de l'Exemple 12 (500 mg; 2,8 mmol), le diéthylcyanophosphonate (2 éq; 8,4 mmol.; 16,8 ml) sont mélangés dans 25 ml de THF. Après 30 minutes d'agitation, le milieu réactionnel est hydrolysé, puis extrait à l'AcOEt. En parallèle, une solution d'iodure de samarium est préparée. Le samarium (4,5 éq; 12,6 mmol.; 1,9 g) est mis en suspension dans 15 ml de THF, puis goutte à goutte le diiodoéthane (3 éq; 8,4 mmol.; 2,37 g), dilué dans 15 ml de THF, est additionné. Lorsque la solution d'iodure de samarium est devenue bleue, le complexe formé précédemment est dissous dans 5 ml de THF et 0,3 ml de *tert*-butanol, puis additionné à cette solution de SmI₂. La solution est laissée sous agitation pendant 12 heures à température ambiante. Le milieu réactionnel est hydrolysé avec une solution d'HCl à 10 %. Après extraction à l'AcOEt, la phase organique est lavée avec une solution de Na₂S₂O₃ à 10 %, puis deux fois avec une solution saturée de NaHCO₃.
Le résidu obtenu est purifié par chromatographie "flash" sur gel de silice (éluant : EP/AcOEt (8/2)). Le produit du titre est obtenu sous forme d'huile.

### Stade B : N-[(6-Méthoxy-3,4-dihydro-2H-4-chroményl)méthyl]acétamide

Le nitrile obtenu au stade A (200 mg; 1,05 mmol), est solubilisé dans le réacteur de l'appareil de Parr dans 15 ml d'anhydride acétique. Le nickel de Raney (60 mg; 30 % en masse), ainsi que l'acétate de sodium (1,5 éq; 1,58 mmol.; 130 mg) sont ajoutés. On place le réacteur sous une pression d'hydrogène de 580 Psi, à 50°C pendant 12 heures. Lorsque la réaction est terminée, on filtre sur célite, puis on rince à l'AcOEt. Le produit est purifié par chromatographie "flash" sur gel de silice (éluant : AcOEt (100 %)). Le solide blanc obtenu est recristallisé dans un mélange pentane-éther.
Point de fusion : 140-141°C

| Microanalyse Elémentaire : | | | |
|---|---|---|---|
| | C | H | N |
| %Calculé | 66,36 | 7,28 | 5,95 |
| % Trouvé | 66,43 | 7,40 | 5,95 |

### EXEMPLE 47 : N-[(6-Métboxy-3,4-dihydro-2H-4-chroményl)méthyl]butanamide

En milieu anhydre, à 0°C, le nitrile obtenu au stade A de l'Exemple 46 (500 mg; 2,64 mmol), est solubilisé dans 5 ml d'éther, puis LiAlH₄ (126 mg; 5,5 mmol.; 2,1 éq) est additionné par portions. Le mélange est porté à reflux pendant 2 heures. L'hydrolyse du milieu réactionnel est réalisée au moyen de 130 µl d'eau, 130 µl d'une solution de NaOH 15 %, puis avec 370 µl d'eau. Les sels sont filtrés sur fritté, rincés au CH₂Cl₂ puis les solvants sont éliminés sous pression réduite. L'amine ainsi obtenue (300 mg ; 1,53 mmol.) est dissoute dans 6 ml de CH₂Cl₂, à 0°C. La triéthylamine (3 éq; 4,59 mmol.; 640 µl) et le chlorure de butyryle (1,4 éq; 2,14 mmol.; 222 µl) sont ajoutés. On laisse sous agitation, à température ambiante, pendant 5 heures. Après évaporation du solvant, le produit est extrait au CH₂Cl₂. Le résidu est purifié par chromatographie "flash" sur gel de silice (éluant AcOEt/EP (1/1)). Le produit du titre est obtenu sous la forme d'une huile.

| Microanalyse Elémentaire : | | | |
|---|---|---|---|
| | C | H | N |
| % Calculé | 68,41 | 8,04 | 5,32 |
| % Trouvé | 68,42 | 8,23 | 5,15 |

### EXEMPLE 48 : N-[2-(6-Méthoxy-3,4-dihydro-2H-4-chroményl)éthyl]-3-butènamide

En milieu anhydre, le nitrile obtenu au stade E de l'Exemple 12 (740 mg; 3,64 mmol), est solubilisé dans 30 ml d'éther, puis LiAlH₄ (1,5 éq; 5,46 mmol.; 207 mg) est additionné par portions. On laisse 3 heures à reflux, sous argon. Après retour à température ambiante, on ajoute successivement 210 µl d'eau, 210 µl d'une solution de NaOH 15 %, puis 620 µl d'eau. La solution est laissée 30 minutes à température ambiante, sous agitation. Les sels sont filtrés sur fritté, le filtrat séché sur MgSO₄ et évaporé sous pression réduite. L'huile jaune obtenue est directement engagée dans l'étape suivante : en milieu anhydre, à -10°C, l'acide 3-butènoïque (1,5 éq; 5,43 mmol; 467 mg) et l'HOBT (1,5 éq; 5,43 mmol; 733 mg). On laisse sous agitation, sous argon, à -10°C pendant 30 minutes. En parallèle, l'amine est dissoute dans 10 ml de CH₂Cl₂ distillé et mise en présence de triéthylamine (1 éq; 3,62 mmol; 504 µl). L'acide 3- butènoïque "activé" est ensuite additionné à la solution d'amine libre. Le milieu réactionnel est laissé 2 heures à -10°C, puis 60 heures à température ambiante. Le mélange est ensuite successivement lavé avec HCl 1N, à l'eau, avec NaOH 1N, à l'eau, séché sur MgSO₄, et le solvant évaporé sous pression réduite. Le résidu obtenu est purifié par chromatographie "flash" sur gel de silice (éluant EP/AcOEt (3/7)). Le produit est obtenu sous la forme de cristaux blancs, après lavage à l'éther.
Point de fusion : 73-74°C

| Microanalyse Elémentaire : | | | |
|---|---|---|---|
| | C | H | N |
| % Calculé | 69,79 | 7,69 | 5,09 |
| % Trouvé | 69,57 | 7,67 | 5,17 |

### EXEMPLE 50 : N-[(6-Méthoxy-3,4-dihydro-2H-3-chroményl)méthyl]butanamide

### Stade A : 6-Méthoxy-3-chromanecarbonitrile

0,9 g (4,8 mmol) du nitrile obtenu dans la Préparation 11 est ajouté à une suspension de 0,09 g de palladium/charbon au sein de 15 ml de méthanol anhydre puis le mélange est soumis à une pression d'hydrogène de 50 psi pendant 22 heures. Après filtration du catalyseur résiduel, le solvant est évaporé sous vide puis le brut réactionnel est purifié sur colonne de silice (éluant : éther de pétrole/acétate d'éthyle 75/25) pour conduire au nitrile du titre sous forme d'un solide blanc.
Point de fusion : 79-80°C

### Stade B : (6-Méthoxy-3,4-dihydro-2H-3-chroményl)méthylamine

0,51 g (2,7 mmol) du nitrile obtenu au stade A est réduit à l'aide de l'hydrure de lithium aluminium au sein de l'éther pour conduire à l'amine du titre sous forme d'une huile jaune.

### Stade C : N-[(6-Méthoxy-3,4-dihydro-2H-3-chroményl)méthyl]butanamide

Une solution de 0,4 g (2,06 mmol) de l'amine obtenue au stade B au sein de 20 ml de dichlorométhane anhydre est refroidie à 0°C. 1 ml (7,2 mmol) de triéthylamine ainsi que 0,38 g (3,6 mmol) de chlorure de butyryle sont successivement additionnés au milieu. Après une heure d'agitation sous argon le mélange réactionnel est acidifié à l'aide d'une solution d'acide chlorhydrique 1N puis la phase aqueuse est extraite au dichlorométhane. La phase organique est séchée sur sulfate de magnésium puis concentrée sous vide. L'amide du titre est obtenue pur sous forme d'un solide blanc après passage sur colonne de silice (éluant : éther de pétrole/acétate d'éthyle 50/50).
Point de fusion : 110-111°C

| Microanalyse Elémentaire : | | | |
|---|---|---|---|
| | C | H | N |
| % Calculé | 68,41 | 8,04 | 5,32 |
| % Trouvé | 68,59 | 8,13 | 5,39 |

### EXEMPLE 51 : N-[2-(9-Méthoxy-2,3-dihydro-1H-benzo[f]chromèn-1-yl)éthyl] Acétamide

Le nitrile obtenu dans la Préparation 16 (400 mg; 1,58 mmol) est solubilisé dans le réacteur de Parr dans 22 ml d'anhydride acétique. Le nickel de Raney (60 mg; 30 % en mass), ainsi que l'acétate de sodium (1,5 éq; 2,37 mmol.; 208 mg) sont ajoutés. On place le réacteur sous une pression d'hydrogène de 50 Psi. Après 12 heures de réaction à 50°C, le mélange réactionnel est filtré sur célite, puis rincé au CH₂Cl₂. Les différents solvants sont évaporés puis le résidu est hydrolysé et extrait à l'AcOEt. Le produit est purifié par chromatographie "flash" sur gel de silice (éluant : AcOEt (100 %) puis AcOEt/MeOH (95/5)). Le solide est obtenu après recristallisation dans l'éther contenant quelques gouttes d'isopropanol.
Point de fusion : 106-107°C

| Microanalyse Elémentaire : | | | |
|---|---|---|---|
| | C | H | N |
| % Calculé | 72,21 | 7,07 | 4,68 |
| % Trouvé | 71,81 | 7,22 | 4,70 |

### EXEMPLE 52 : N-[2-(9-Méthoxy-2,3-dihydro-1H-benzo[f]chromèn-1-yl)éthyl] Butanamide

En milieu anhydre et à 0°C, le nitrile obtenu dans la Préparation 16 (200 mg; 0,79 mmol), est solubilisé dans 5 ml d'éther, puis LiAlH₄ (2,1 éq; 1,66 mmol.; 63 mg) est ajouté par portions. Le mélange est porté à reflux pendant 2 heures. L'hydrolyse du milieu se fait avec 64 µl d'eau, 64 µl d'une solution de NaOH 15 %, puis 190 µl d'eau. Les sels sont filtrés sur fritté, rincés avec de l'éther, puis les solvants sont éliminés sous pression réduite. L'amine ainsi obtenue (0,79 mmol) est dissoute dans 2 ml de CH₂Cl₂, à 0°C. La triéthylamine (3 éq; 1,87 mmol.; 260 µl) est ajoutée ainsi que le chlorure de butyryle (1,4 éq; 1,11 mmol.; 115 µl). On laisse sous agitation à température ambiante pendant 2 heures. Après évaporation du solvant, le produit est extrait au CH₂Cl₂. Le résidu est purifié par chromatographie "flash" sur gel de silice (éluant CH₂Cl₂ (100 %) puis CH₂Cl₂/MeOH (95/5)). Le produit du titre est obtenu sous la forme d'un solide après recristallisation dans le mélange Et₂O/EtOH (8/2).
Point de fusion: 114°C

| Microanalyse Elémentaire : | | | |
|---|---|---|---|
| | C | H | N |
| % Calculé | 73,36 | 7,70 | 4,28 |
| % Trouvé | 73,00 | 7,56 | 4,18 |

### EXEMPLE 53 : N-[2-(9-Méthoxy-2,3-dihydro-1H-benzo[f]chromèn-1-yl)méthyl] acétamide

Le nitrile obtenu dans la Préparation 15 (200 mg; 0,84 mmol) est solubilisé dans le réacteur de Parr dans 15 ml d'anhydride acétique. Le nickel de Raney (60 mg; 30 % en masse), ainsi que l'acétate de sodium (3 éq; 2,52 mmol.; 200 mg) sont ajoutés. On place le réacteur sous une pression d'hydrogène de 50 Psi. Après 12 heures de réaction à 50°C, le mélange réactionnel est filtré sur célite, puis rincé au CH₂Cl₂. Les différents solvants sont évaporés puis le résidu est hydrolyse et extrait à l'AcOEt. Le produit est purifié par chromatographie "flash" sur gel de silice (éluant : CH₂Cl₂/MeOH (95/5)). Le composé du titre est obtenu sous forme de solide après lavage à l'éther.
Point de fusion : 145-147°C

| Microanalyse Elémentaire : | | | |
|---|---|---|---|
| | C | H | N |
| % Calculé | 71,55 | 6,71 | 4,91 |
| % Trouvé | 71,84 | 6,66 | 5,04 |

### EXEMPLE 54 : N-[2-(9-Méthoxy-2,3-dihydro-1H-benzo[f]chromèn-1-yl)méthyl] butanamide

En milieu anhydre et à 0°C, le nitrile obtenu dans la Préparation 15 (300 mg; 1,25 mmol), est solubilisé dans 5 ml d'éther, puis LiAlH₄ (3 éq; 3,75 mmol.; 143 mg) est ajouté par portions. Le mélange est porté à reflux pendant 3 heures. L'hydrolyse du milieu se fait avec 220 µl d'eau, 64 µl d'une solution de NaOH 15 %, puis 650 µl d'eau. Les sels sont filtrés sur fritté, rincés au CH₂Cl₂, puis les solvants sont éliminés sous pression réduite. L'amine ainsi obtenue (300 mg ; 1,53 mmol) est dissoute dans 6 ml de CH₂Cl₂, à O°C. La triéthylamine (3 éq; 3,76 mmol.; 254 µl) est ajoutée ainsi que le chlorure de butyryle (1,4 éq; 1,76 mmol.; 183 µl). On laisse sous agitation à température ambiante pendant 5 heures. Après évaporation du solvant, le produit est extrait au CH₂Cl₂. Le résidu est purifié par chromatographie "flash" sur gel de silice (éluant AcOEt/EP (7/3)). Le produit du titre est obtenu sous la forme d'un solide blanc après cristallisation dans l'éther.
Point de fusion : 118-119°C

| Microanalyse Elémentaire : | | | |
|---|---|---|---|
| | C | H | N |
| % Calculé | 72,81 | 7,40 | 4,47 |
| % Trouvé | 72,49 | 7,40 | 4,71 |

### ETUDE PHARMACOLOGIQUE

### EXEMPLE A : Etude de la toxicité aiguë

La toxicité aiguë a été appréciée après administration orale à des lots de 8 souris (26±2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité de la plupart des composés de l'invention.

### EXEMPLE B : Etude de liaison aux récepteurs de la mélatonine

### 1) Etude sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I] - iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### 2) Etude sur des membranes d'homogénat de cerveau de poulet (Gallus Domesticus)

Les animaux utilisés sont des poulets (Gallus domesticus) âgés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology, 128, pp 475-482, 1991). La 2-[¹²⁵I] iodomélatonine est incubée en présence des membranes dans une solution tamponnée à pH 7,4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman^{®} LS 6000.

Les produits utilisés sont :
- 2[¹²⁵I] iodo mélatonine
- mélatonine
- produits courants
- molécules originales

En screening primaire, les molécules sont testées à 2 concentrations (10⁻⁷ et 10⁻⁵ M). Chaque résultat est la moyenne de 3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention montrent que la liaison des composés testés est très puissante.

### EXEMPLE C : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques. Les effets des molécules sont testés sur de nombreux paramètres et, en particulier, sur les rythmes circadiens d'activité locomotrice qui représentent un marqueur fiable de l'activité de l'horloge circadienne endogène.
Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (L/D 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes circadiens.
Dès que les rythmes enregistrés témoignent d'un entraînement stable par le cycle lumineux L/D 12 : 12, les rats sont mis en obscurité permanente (D/D).
Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le cycle lumière/obscurité (L/D),
- disparition de l'entraînement des rythmes en obscurité permanente,
- entraînement de l'activité par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE E : Activité Antiarythmique

### Protocole

### (Ref: LAWSON J.W. et al. J. Pharmacol. Expert. Therap., 1968, 160, pp 22-31)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : Composition pharmaceutique : comprimés

| | |
|---|---|
| 1000 comprimés dosés à 5 mg de N-(9-Méthoxy-2,3-dihydro-1*H*-benzo[f] | |
| chromèn-2-yl)acétamide (Exemple 5) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composé de formule (I) : 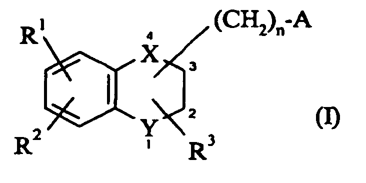 dans laquelle
◆ R¹ représente un groupement OR⁴ dans lequel R⁴ représente un atome d'hydrogène, un groupement alkyle (C₁-C₆) linéaire ou ramifié substitué ou non, alcényle (C₂-C₆) linéaire ou ramifié substitué ou non, alcynyle (C₂-C₆) linéaire ou ramifié substitué ou non, aryle, arylalkyle (C₁-C₆) linéaire ou ramifié, cycloalkyle (C₃-C₈) substitué ou non, ou cycloalkyl (C₃-C₈) alkyle (C₁-C₆) linéaire ou ramifié substitué ou non,
◆ R² représente un atome d'hydrogène,
ou R¹ et R², situés sur deux carbones adjacents, forment ensemble avec les atomes de carbone qui les portent, un groupement phényle ou phényle substitué,
◆ X représente un groupement CH ou CH₂,
◆ Y représente un atome d'oxygène,
◆ R³ représente un atome d'hydrogène, un groupement aryle, arylalkyle (C₁-C₆) linéaire ou ramifié, ou alkyle (C₁-C₆) linéaire ou ramifié,
◆ n vaut 0, 1, 2, 3, 4 ou 5 lorsque R¹ et R², situés sur deux carbones adjacents, forment ensemble, avec les atomes de carbone qui les portent un groupement phényle ou phényle substitué,
ou n vaut 1, 2, 3, 4 ou 5 lorsque R² représente un atome d'hydrogène,
◆ A représente
• un groupement NR⁵R⁶ dans lequel
R⁶ représente un atome d'hydrogène ou un groupement alkyle (C₁-C₆) linéaire ou ramifié,
R⁵ représente un groupement 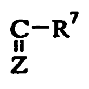 dans lequel Z représente un atome d'oxygène ou un atome de soufre, et R⁷ représente :
- un atome d'hydrogène,
- un groupement R⁸ représentant un groupement alkyle (C₁-C₆) linéaire ou ramifié substitué ou non, cycloalkyle (C₃-C₈) substitué ou non, cycloalkyl (C₃-C₈) alkyle (C₁-C₆) linéaire ou ramifié substitué ou non, alcényle (C₂-C₆) linéaire ou ramifié substitué ou non, alcynyle (C₂-C₆) linéaire ou ramifié substitué ou non, aryle, ou arylalkyle (C₁-C₆) linéaire ou ramifié,
- ou un groupement NR⁸R⁹ dans lequel R⁹ représente un atome d'hydrogène ou un groupement alkyle (C₁-C₆) linéaire ou ramifié et R⁸ est tel que défini précédemment,
• ou un groupement 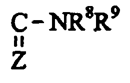 dans lequel Z, R⁸ et R⁹ sont tels que définis précédemment,
étant entendu que :
- le terme "substitué" affecté au terme "phényle" signifie que ce groupement est substitué par un ou plusieurs atomes d'halogène, ou un ou plusieurs groupements, identiques ou différents, choisis parmi, OH, alkoxy (C₁-C₆) linéaire ou ramifié, alkyle (C₁-C₆) linéaire ou ramifié, cyano, nitro, amino, alkylamino, dialkylamino ou trihalogénoalkyle,
- le terme "substitué" affecté aux termes "alkyle", "alcényle" et "alcynyle" signifie que ce groupement est substitué par un ou plusieurs atomes d'halogène, ou un ou plusieurs groupements, identiques ou différents, choisis parmi OH, alkoxy (C₁-C₆) linéaire ou ramifié, amino, alkylamino ou dialkylamino,
- le terme "substitué" affecté aux termes "cycloalkyle" et "cycloalkylalkyle" signifie que la partie cyclique est substituée par un ou plusieurs atomes d'halogène, ou un ou plusieurs groupements, identiques ou différents, choisis parmi alkyle (C₁-C₆) linéaire ou ramifié, alkoxy (C₁-C₆) linéaire ou ramifié, hydroxy, oxo, amino, alkylamino ou dialkylamino,
étant entendu que :
- lorsque le composé de formule (I) est un dérivé chromane (X représente un groupement CH₂, Y représente un atome d'oxygène et R¹, R² et R³ sont tels que définis précédemment), alors le groupement -(CH₂)ₙ-A est différent des groupements suivants :
- CH₂-NHCORₑ (où Rₑ représente un groupement cycloalkyle) en position 2 du noyau chromane,
- CH₂-CONHR_{f} (où R_{f} représente un groupement benzyle ou 1-phényl-2-hydroxyéthyle) en position 4 du noyau chromane,
- lorsque A représente un groupement NHCSNHR⁸ et n vaut 2, alors R⁸ ne peut représenter un groupement aryle,
- lorsque X représente un groupement CH₂, R¹ est tel que défini précédemment et R² représente un atome d'hydrogène, alors A ne peut représenter un groupement urée ou thiourée substituée par un noyau phényle (substitué ou non),
leurs énantiomères et diastéréoisomères, ainsi que leurs sels d'addition à un acide ou une base pharmaceutiquement acceptable.

2. Composés de formule (I) selon la revendication 1 pour lesquels R¹ et R² situés sur deux carbones adjacents forment ensemble avec les atomes de carbone qui les portent un groupement phényle ou phényle substitué, leurs énantiomères et diastéréoisomères, ainsi que leurs sels d'addition à un acide ou une base pharmaceutiquement acceptable.

3. Composés de formule (I) selon la revendication 1 pour lesquels R¹ représente un groupement OR⁴, leurs énantiomères et diastéréoisomères, ainsi que leurs sels d'addition à un acide ou une base pharmaceutiquement acceptable.

4. Composés de formule (I) selon la revendication 1 pour lesquels R³ représente un atome d'hydrogène, leurs énantiomères et diastéréoisomères, ainsi que leurs sels d'addition à un acide ou une base pharmaceutiquement acceptable.

5. Composés de formule (I) selon la revendication 1 pour lesquels R³ représente un groupement aryle, leurs énantiomères et diastéréoisomères, ainsi que leurs sels d'addition à un acide ou une base pharmaceutiquement acceptable.

6. Composés de formule (I) selon la revendication 1 pour lesquels A représente un groupement de formule NR⁵R⁶, leurs énantiomères et diastéréoisomères, ainsi que leurs sels d'addition à un acide ou une base pharmaceutiquement acceptable.

7. Composés de formule (I) selon la revendication 1 qui sont des dihydrobenzochromènes ou dihydrochromènes, leurs énantiomères et diastéréoisomères, ainsi que leurs sels d'addition à un acide ou une base pharmaceutiquement acceptable.

8. Composés de formule (I) selon la revendication 1 qui est le N-(9-Méthoxy-2,3-dihydro-1*H*-benzo[f]chromèn-2-yl)acétamide, ses énantiomères et diastéréoisomères, ainsi que ses sels d'addition à un acide ou une base pharmaceutiquement acceptable.

9. Composés de formule (I) selon la revendication 1 qui est le N-[2-(6-Méthoxy-3,4-dihydro-2*H*-chroményl)éthyl]acétamide, ses énantiomères et diastéréoisomères, ainsi que ses sels d'addition à un acide ou une base pharmaceutiquement acceptable.

10. Composés de formule (I) selon la revendication 1 qui est le N-[(6-Méthoxy-2*H*-3-chroményl)méthyl]butanamide, ses énantiomères et diastéréoisomères, ainsi que ses sels d'addition à un acide ou une base pharmaceutiquement acceptable.

11. Procédé de préparation des composés de formule (I) selon la revendication 1 **caractérisé en ce que** l'on prend comme produit de départ un composé de formule (II): 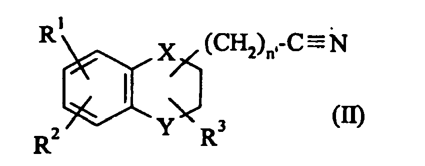 dans laquelle R¹, R², R³, X et Y sont tels que définis précédemment, et n' peut prendre les valeurs de 0 à 4,
que l'on soumet :
◆ à un agent réducteur pour conduire au composé de formule (III) : 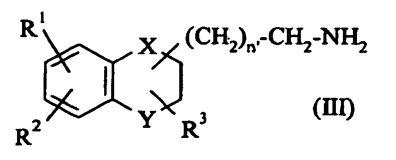 dans laquelle R¹, R², R³, X, Y et n' sont tels que définis précédemment,
les composés de formule (III) pouvant par ailleurs être obtenus
- par réduction du composé de formule (IV) : 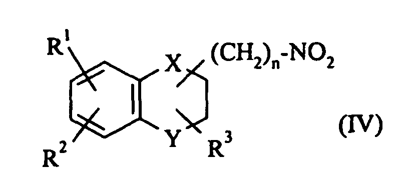
dans laquelle R¹, R², R³, X, Y et n sont tels que définis précédemment,
- ou à partir du composé de formule (V) : 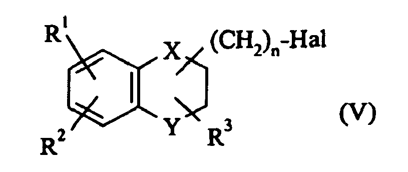
dans laquelle R¹, R², R³, X, Y et n sont tels que définis précédemment et Hal représente un atome d'halogène,
que l'on substitue par un groupement phtalimide puis que l'on soumet à une hydrazinolyse,
composé de formule (III) sur lequel on condense :
- soit un chlorure d'acyle CICOR⁸ ou l'anhydride d'acide (mixte ou symétrique) correspondant pour lesquels R⁸ est tel que défini précédemment,
pour conduire au composé de formule (I/a), cas particulier des composés de formule (I) : 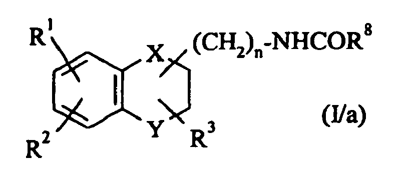 dans laquelle R¹, R², R³, R⁸, X, Y et n sont tels que définis précédemment,
qui peut être soumis à un agent de thionation comme le réactif de Lawesson pour obtenir le composé de formule (I/b), cas particulier des composés de formule (I): 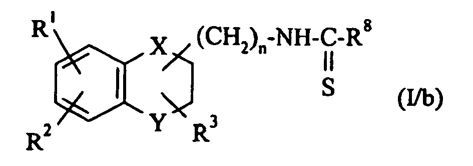 dans laquelle R¹, R², R³, R⁸, X, Y et n sont tels que définis précédemment,
- soit un composé de formule (VI) :
Z=C=N-R⁸ (VI)
dans laquelle Z et R⁸ sont tels que définis précédemment,
afin d'obtenir le composé de formule (I/c), cas particulier des composés de formule (I): 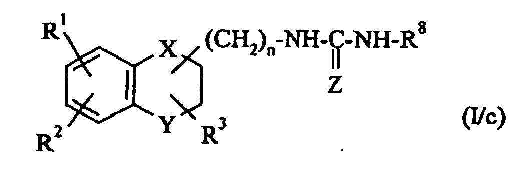
dans laquelle R¹, R², R³, R⁸, X, Y, Z et n sont tels que définis précédemment,
l'ensemble des composés de formule (I/a), (I/b) et (I/c) formant le composé de formule (I/d), cas particulier des composés de formule (I) : 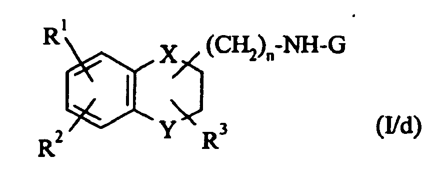 dans laquelle R¹, R², R³, X, Y et n sont tels que définis précédemment, et G représente un groupement COR⁸, CSR⁸ ou CZNHR⁸ dans lesquels Z et R⁸ sont tels que définis précédemment,
que l'on peut alkyler selon une technique classique d'alkylation grâce à un composé de formule (VII) :
Alk-W (VII)
dans laquelle Alk représente un groupement alkyle (C₁-C₆) linéaire ou ramifié et W représente un groupe partant comme un atome d'halogène ou un groupement tosyle,
ou grâce à un dialkylsulfate,
pour conduire au composé de formule (I/e), cas particulier des composés de formule (I) : 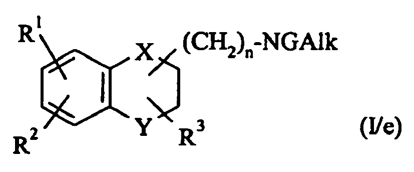 dans laquelle R¹, R², R³, X, Y, G, Alk et n sont tels que définis précédemment,
◆ ou à une hydrolyse en milieu acide ou basique pour conduire au composé de formule (VIII) : 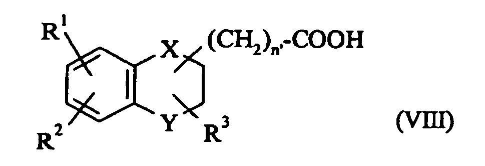
dans laquelle R¹, R², R³, X, Y et n' sont tels que définis précédemment,
qui est soumis, après activation sous forme de chlorure d'acide ou en présence d'agent de couplage, à l'action d'une amine H₂NR⁸ où R⁸ est tel que défini précédemment, pour conduire au composé de formule (I/f), cas particulier des composés de formule (I) : 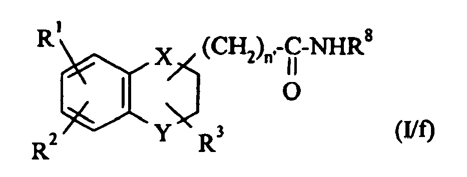 dans laquelle R¹, R², R³, R⁸, X, Y et n' sont tels que définis précédemment,
qui peut être soumis à un agent de thionation comme le réactif de Lawesson pour obtenir le composé (I/g), cas particulier des composés de formule (I) : 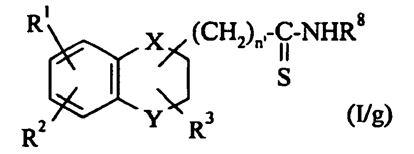 dans laquelle R¹, R², R³, R⁸, X, Y et n' sont tels que définis précédemment,
l'ensemble des composés (I/f) et (I/g) formant le composé de formule (I/h) : 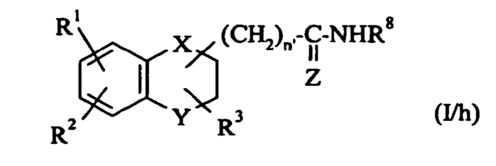 dans laquelle R¹, R², R³, R⁸, X, Y, Z et n' sont tels que définis précédemment,
qui peut être alkylé selon une technique classique d'alkylation pour conduire au composé de formule (I/i) : 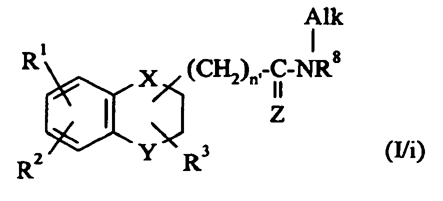 dans laquelle R¹, R², R³, R⁸, X, Y, Z , Alk et n' sont tels que définis précédemment,
les composés de formule (I/a) à (I/i) formant l'ensemble des composés de formule (I) qui peuvent être purifiés selon une technique classique de séparation, que l'on transforme, si on le souhaite en leurs sels d'addition à un acide ou à une base pharmaceutiquement acceptable, et dont on sépare éventuellement les isomères selon une technique classique de séparation.

12. Compositions pharmaceutiques contenant comme principe actif au moins un des composés de formule (I) selon l'une quelconque des revendications 1 à 10 ou un de ses sels d'addition avec un acide ou une base pharmaceutiquement acceptable en combinaison avec un ou plusieurs excipients pharmaceutiquement acceptables.

13. Compositions pharmaceutiques selon la revendication 12 utiles pour le traitement des troubles du système mélatoninergique.

## Claims

1. Compounds of formula (I) : 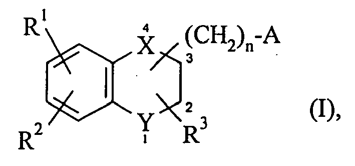 wherein
◆ R¹ represents an OR⁴ group wherein R⁴ represents a hydrogen atom, a substituted or unsubstituted linear or branched (C₁-C₆)alkyl group, a substituted or unsubstituted linear or branched (C₂-C₆)alkenyl group, a substituted or unsubstituted linear or branched (C₂-C₆)alkynyl group, an aryl group, an aryl-(C₁-C₆)alkyl group in which the alkyl moiety is linear or branched, a substituted or unsubstituted (C₃-C₈)cycloalkyl group or a substituted or unsubstituted (C₃-C₈)cycloalkyl-(C₁-C₆)alkyl group in which the alkyl moiety is linear or branched,
◆ R² represents a hydrogen atom,
or R¹ and R², located on two adjacent carbon atoms, form together with the carbon atoms that carry them a phenyl or substituted phenyl group,
◆ X represents a group CH or CH₂,
◆ Y represents an oxygen atom,
◆ R³ represents a hydrogen atom, an aryl group, an aryl-(C₁-C₆)alkyl group in which the alkyl moiety is linear or branched, or a linear or branched (C₁-C₆)alkyl group,
◆ n is equal to 0, 1, 2, 3, 4 or 5 when R¹ and R², located on two adjacent carbon atoms, form together with the carbon atoms that carry them a phenyl or substituted phenyl group,
or n is equal to 1, 2, 3, 4 or 5 when R² represents a hydrogen atom,
◆ A represents
• an NR⁵R⁶ group wherein
R⁶ represents a hydrogen atom or a linear or branched (C₁-C₆)alkyl group,
R⁵ represents a 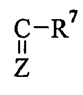 group wherein Z represents an oxygen atom or a sulphur atom, and R⁷ represents :
- a hydrogen atom,
- an R⁸ group which represents a substituted or unsubstituted linear or branched (C₁-C₆)alkyl group, a substituted or unsubstituted (C₃-C₈)-cycloalkyl group, a substituted or unsubstituted (C₃-C₈)cycloalkyl-(C₁-C₆)alkyl group in which the alkyl moiety is linear or branched, a substituted or unsubstituted linear or branched (C₂-C₆)alkenyl group, a substituted or unsubstituted linear or branched (C₂-C₆)alkynyl group, an aryl group or an aryl-(C₁-C₆)alkyl group in which the alkyl moiety is linear or branched,
- or an NR⁸R⁹ group wherein R⁹ represents a hydrogen atom or a linear or branched (C₁-C₆)alkyl group and R⁸ is as defined hereinabove,
• or a 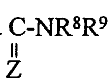 group wherein Z, R⁸ and R⁹ are as defined hereinabove, it being understood that:
- the term "substituted" used in respect of the term "phenyl" means that the group is substituted by one or more halogen atoms or one or more identical or different groups selected from OH, linear or branched (C₁-C₆)alkoxy, linear or branched (C₁-C₆)alkyl, cyano, nitro, amino, alkylamino, dialkylamino and trihaloalkyl,
- the term "substituted" used in respect of the terms "alkyl", "alkenyl" and "alkynyl" means that the group is substituted by one or more halogen atoms or one or more identical or different groups selected from OH, linear or branched (C₁-C₆)alkoxy, amino, alkylamino and dialkylamino,
- the term "substituted" used in respect of the terms "cycloalkyl" and "cycloalkylalkyl" means that the cyclic moiety is substituted by one or more halogen atoms or one or more identical or different groups selected from linear or branched (C₁-C₆)alkyl, linear or branched (C₁-C₆)alkoxy, hydroxy, oxo, amino, alkylamino and dialkylamino,
provided that :
- when the compound of formula (I) is a chroman compound (X represents a group CH₂, Y represents an oxygen atom, and R¹, R² and R³ are as defined hereinabove), the group -(CH₂)ₙ-A is other than the following groups :
- CH₂-NHCORₑ (wherein Rₑ represents a cycloalkyl group) in the 2-position of the chroman group,
- CH₂-CONHR_{f} (wherein R_{f} represents a benzyl or 1-phenyl-2-hydroxyethyl group) in the 4-position of the chroman group,
- when A represents a NHCSNHR⁸ group and n is equal to 2, R⁸ cannot represent an aryl group,
- when X represents a CH₂ group, R¹ is as defined hereinabove and R² represents a hydrogen atom, A cannot represent a urea or thiourea group substituted by a substituted or unsubstituted phenyl group,
their enantiomers and diastereoisomers, and addition salts thereof with a pharmaceutically acceptable acid or base.

2. Compounds of formula (I) according to claim 1 wherein R¹ and R², located on two adjacent carbon atoms, form together with the carbon atoms that carry them a phenyl or substituted phenyl group, their enantiomers and diastereoisomers, and addition salts thereof with a pharmaceutically acceptable acid or base.

3. Compounds of formula (I) according to claim 1 wherein R¹ represents an OR⁴ group, their enantiomers and diastereoisomers, and addition salts thereof with a pharmaceutically acceptable acid or base.

4. Compounds of formula (I) according to claim 1 wherein R³ represents a hydrogen atom, their enantiomers and diastereoisomers, and addition salts thereof with a pharmaceutically acceptable acid or base.

5. Compounds of formula (I) according to claim 1 wherein R³ represents an aryl group, their enantiomers and diastereoisomers, and addition salts thereof with a pharmaceutically acceptable acid or base.

6. Compounds of formula (I) according to claim 1 wherein A represents a group of formula NR⁵R⁶, their enantiomers and diastereoisomers, and addition salts thereof with a pharmaceutically acceptable acid or base.

7. Compounds of formula (I) according to claim 1 which are dihydrobenzochromenes or dihydrochromenes, their enantiomers and diastereoisomers, and addition salts thereof with a pharmaceutically acceptable acid or base.

8. Compound of formula (I) according to claim 1 which is N-(9-methoxy-2,3-dihydro-1*H*-benzo[f]chromen-2-yl)acetamide, its enantiomers and diastereoisomers, and addition salts thereof with a pharmaceutically acceptable acid or base.

9. Compound of formula (I) according to claim 1 which is N-[2-(6-methoxy-3,4-dihydro-2*H*-chromenyl)ethyl]acetamide, its enantiomers and diastereoisomers, and addition salts thereof with a pharmaceutically acceptable acid or base.

10. Compound of formula (I) according to claim 1 which is N-[(6-methoxy-2*H*-3-chromenyl)methyl]butanamide, its enantiomers and diastereoisomers, and addition salts thereof with a pharmaceutically acceptable acid or base.

11. Process for the preparation of compounds of formula (I) according to claim 1, **characterised in that** there is used as starting material a compound of formula (II) : 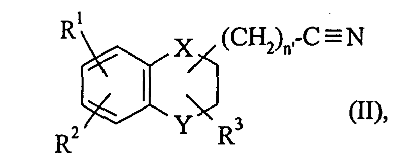 wherein R¹, R², R³, X and Y are as defined hereinabove, and n' can have the values from 0 to 4,
which is subjected:
◆ to a reducing agent to yield a compound of formula (III) : 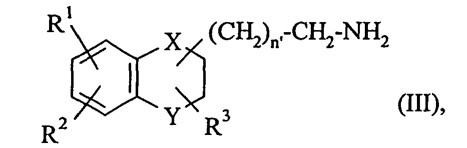 wherein R¹, R², R³, X, Y and n' are as defined hereinabove,
which compounds of formula (III) can, moreover, be obtained
- by reduction of a compound of formula (IV) : 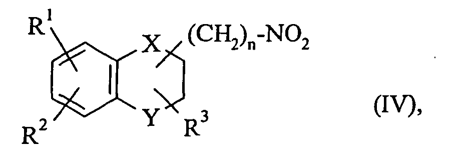 wherein R¹, R², R³, X, Y and n are as defined hereinabove,
- or starting from a compound of formula (V) : 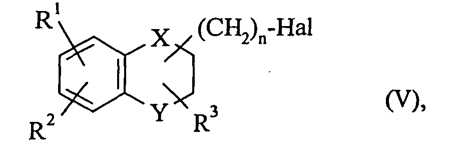 wherein R¹, R², R³, X, Y and n are as defined hereinabove and Hal represents a halogen atom,
which is substituted by a phthalimide group and is then subjected to hydrazinolysis,
with which compound of formula (III) there is condensed :
- either an acyl chloride ClCOR⁸ or the corresponding acid anhydride (mixed or symmetrical) wherein R⁸ is as defined hereinabove,
to yield a compound of formula (I/a), which is a particular case of the compounds of formula (I) : 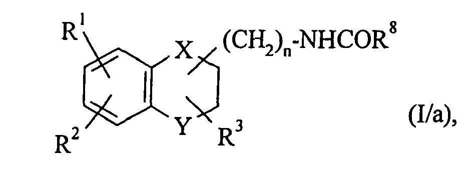
wherein R¹, R², R³, R⁸, X, Y and n are as defined hereinabove,
which can be subjected to a thionisation agent, such as Lawesson's reagent, to obtain a compound of formula (I/b), which is a particular case of the compounds of formula (I) : 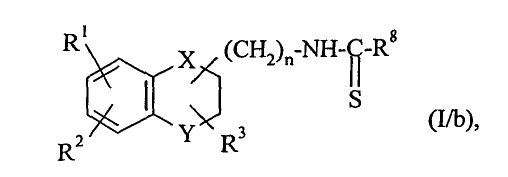 wherein R¹, R², R³, R⁸, X, Y and n are as defined hereinabove,
- or a compound of formula (VI) :
Z=C=N-R⁸ (VI),
wherein Z and R⁸ are as defined hereinabove,
in order to obtain a compound of formula (I/c), which is a particular case of the compounds of formula (I) : 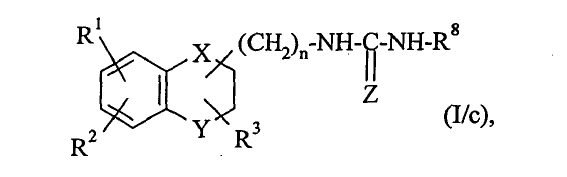
wherein R¹, R², R³, R⁸, X, Y, Z and n are as defined hereinabove,
the totality of the compounds of formulae (I/a), (I/b) and (I/c) constituting the compound of formula (I/d), which is a particular case of the compounds of formula (I): 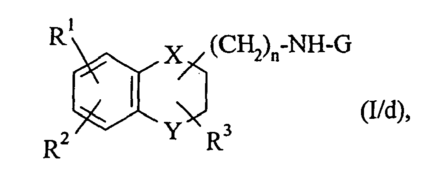 wherein R¹, R², R³, X, Y and n are as defined hereinabove, and G represents a COR⁸, CSR⁸ or CZNHR⁸ group wherein Z and R⁸ are as defined hereinabove,
which can be alkylated in accordance with a conventional alkylation technique using a compound of formula (VII):
Alk-W (VII),
wherein Alk represents a linear or branched (C₁-C₆)alkyl group and W represents a leaving group, such as a halogen atom or a tosyl group,
or using a dialkyl sulphate,
to yield a compound of formula (I/e), which is a particular case of the compounds of formula (I) : 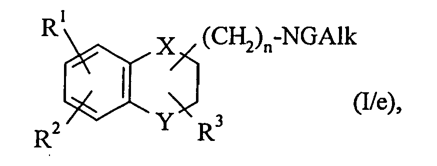 wherein R¹, R², R³, X, Y, G, Alk and n are as defined hereinabove,
◆ or to hydrolysis in an acidic or basic medium to yield a compound of formula (VIII): 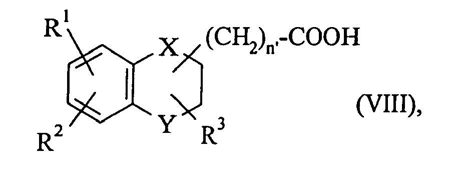
wherein R¹, R², R³, X, Y and n' are as defined hereinabove,
which is subjected, after activation to the acid chloride form or in the presence of a coupling agent, to the action of an amine H₂NR⁸ wherein R⁸ is as defined hereinabove, to yield a compound of formula (I/f), which is a particular case of the compounds of formula (I) : 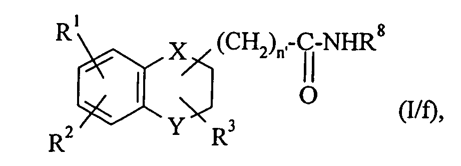 wherein R¹, R², R³, R⁸, X, Y and n' are as defined hereinabove,
which can be subjected to a thionisation agent, such as Lawesson's reagent, to obtain a compound (I/g), which is a particular case of the compounds of formula (I) : 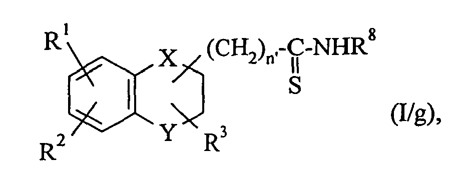 wherein R¹, R², R³, R⁸, X, Y and n' are as defined hereinabove,
the totality of the compounds (I/f) and (I/g) constituting the compound of formula (I/h) : 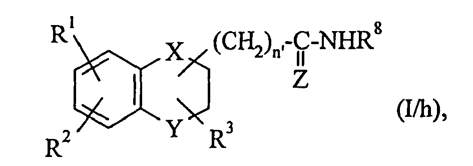 wherein R¹, R², R³, R⁸, X, Y, Z and n' are as defined hereinabove,
which can be alkylated in accordance with a conventional alkylation technique to yield a compound of formula (I/i) : 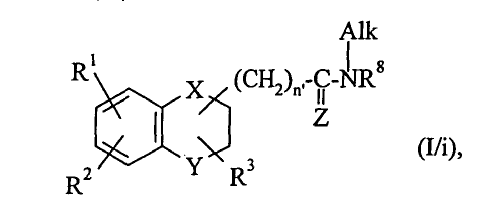 wherein R¹, R², R³, R⁸, X, Y, Z, Alk and n' are as defined hereinabove,
the compounds of formulae (I/a) to (I/i) constituting the totality of the compounds of formula (I), which can be purified in accordance with a conventional separation technique, are converted, if desired, into their addition salts with a pharmaceutically acceptable acid or base and, optionally, are separated into their isomers in accordance with a conventional separation technique.

12. Pharmaceutical compositions comprising as active ingredient at least one compound of formula (I) according to any one of claims 1 to 10 or an addition salt thereof with a pharmaceutically acceptable acid or base in combination with one or more pharmaceutically acceptable excipients.

13. Pharmaceutical compositions according to claim 12 for use in the treatment of disorders of the melatoninergic system.

## Patentansprüche

1. Verbindung der Formel (I): 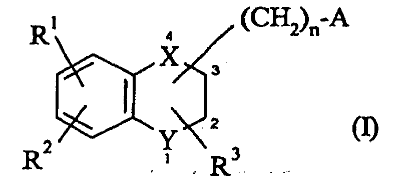 in der:
◆ R¹ eine Gruppe OR⁴ bedeutet, worin R⁴ ein Wasserstoffatom, eine geradkettige oder verzweigte, substituierte oder unsubstituierte (C₁-C₆)-Alkylgruppe, geradkettige oder verzweigte, substituierte oder unsubstituierte (C₂-C₆)-Alkenylgruppe, geradkettige oder verzweigte, substituierte oder unsubstituierte (C₂-C₆)-Alkinylgruppe, Arylgruppe, geradkettige oder verzweigte Aryl-(C₁-C₆)-alkylgruppe, substituierte oder unsubstituierte (C₃-C₆)-Cycloalkylgruppe oder geradkettige oder verzweigte, substituierte oder unsubstituierte (C₃-C₈)-Cycloalkyl-(C₁-C₆)-alkylgruppe darstellt,
◆ R² ein Wasserstoffatom bedeutet,
oder R¹ und R², die an zwei benachbarten Kohlenstoffatomen angeordnet sind, gemeinsam mit den sie tragenden Kohlenstoffatomen eine Phenylgruppe oder substituierte Phenylgruppe bilden,
◆ X eine Gruppe CH oder CH₂ bedeutet,
◆ Y ein Sauerstoffatom bedeutet,
◆ R³ ein Wasserstoffatom, eine Arylgruppe, eine geradkettige oder verzweigte Aryl-(C₁-C₆)-alkylgruppe oder eine geradkettige oder verzweigte (C₁-C₆)-Alkylgruppe bedeutet,
◆ n 0, 1, 2, 3, 4 oder 5 bedeutet, wenn R¹ und R², die an zwei benachbarten Kohlenstoffatomen vorliegen, gemeinsam mit den sie tragenden Kohlenstoffatomen eine Phenylgruppe oder eine substituierte Phenylgruppe bilden,
oder n 1, 2, 3, 4 oder 5 bedeutet, wenn R² ein Wasserstoffatom darstellt,
◆ A:
• eine Gruppe NR⁵R⁶, worin
R⁶ ein Wasserstoffatom oder eine geradkettige oder verzweigte (C₁-C₆)-Alkylgruppe und
R⁵ eine Gruppe 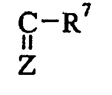 bedeuten, worin Z ein Sauerstoffatom oder ein Schwefelatom darstellt und R⁷:
- ein Wasserstoffatom,
- eine Gruppe R⁸, die für eine geradkettige oder verzweigte, substituierte oder unsubstituierte (C₁-C₆)-Alkylgruppe, substituierte oder unsubstituierte (C₃-C₈)-Cycloalkylgruppe, geradkettige oder verzweigte, substituierte oder unsubstituierte (C₃-C₈)-Cycloalkyl-(C₁-C₆)-alkylgruppe, geradkettige oder verzweigte, substituierte oder unsubstituierte (C₂-C₆)-Alkenylgruppe, geradkettige oder verzweigte, substituierte oder unsubstituierte (C₂-C₆)-Alkinylgruppe, Arylgruppe oder geradkettige oder verzweigte Aryl-(C₁-C₆)-alkylgruppe steht,
- oder eine Gruppe NR⁸R⁹ darstellt, worin R⁹ ein Wasserstoffatom oder eine geradkettige oder verzweigte (C₁-C₆)-Alkylgruppe steht und R⁸ die oben angegebenen Bedeutungen besitzt,
• oder eine Gruppe 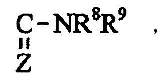 worin Z, R⁸ und R⁹ die oben angegebenen Bedeutungen besitzen, bedeutet,
mit der Maßgabe, daß:
- der Begriff "substituiert" in Bezug auf den Begriff "Phenyl" bedeutet, daß diese Gruppe durch ein oder mehrere Halogenatome oder eine oder mehrere gleichartige oder verschiedenartige Gruppen ausgewählt aus OH, geradkettigem oder verzweigtem (C₁-C₆)-Alkoxy, geradkettigem oder verzweigtem (C₁-C₆)-Alkyl, Cyano, Nitro, Amino, Alkylamino, Dialkylamino oder Trihalogenalkyl substituiert ist,
- der Begriff "substituiert" in Bezug auf die Begriffe "Alkyl", "Alkenyl" und "Alkinyl" bedeutet, daß diese Gruppe durch ein oder mehrere Halogenatome oder eine oder mehrere gleichartige oder verschiedenartige Gruppen ausgewählt aus OH, geradkettigem oder verzweigtem (C₁-C₆)-Alkoxy, Amino, Alkylamino oder Dialkylamino substituiert ist,
- der Begriff "substituiert" in Bezug auf die Begriffe "Cycloalkyl" und "Cycloalkylalkyl" bedeutet, daß der cyclische Teil durch ein oder mehrere Halogenatome oder eine oder mehrere gleichartige oder verschiedenartige Gruppen ausgewählt aus geradkettigem oder verzweigtem (C₁-C₆)-Alkyl, geradkettigem oder verzweigtem (C₁-C₆)-Alkoxy, Hydroxy, Oxo, Amino, Alkylamino oder Dialkylamino substituiert ist,
wobei es sich versteht, daß:
- wenn die Verbindung der Formel (I) ein Chroman-Derivat ist (wobei X eine Gruppe CH₂ bedeutet, Y ein Sauerstoffatom darstellt und R¹, R² und R³ die oben angegebenen Bedeutungen besitzen), dann die Gruppe -(CH₂)ₙ-A von den folgenden Gruppen verschieden ist:
- CH₂-NHCORₑ (worin Rₑ eine Cycloalkylgruppe darstellt) in der 2-Stellung des Chromkerns,
- CH₂-CONHR_{f} (worin R_{f} eine Benzylgruppe oder 1-Phenyl-2-hydroxyethylgruppe bedeutet) in der 4-Stellung des Chromankerns,
- wenn A eine Gruppe NHCSNHR⁸ und n den Wert 2 besitzt, dann R⁸ keine Arylgruppe bedeuten kann,
- wenn X eine Gruppe CH₂ darstellt, R¹ die oben angegebenen Bedeutungen besitzt und R² ein Wasserstoffatom darstellt, dann A keine Harnstoffgruppe oder Thioharnstoffgruppe bedeuten kann, die durch einen (substituierten oder nichtsubstituierten) Phenylkern substituiert ist,
deren Enantiomere und Diastereoisomere sowie deren Additionssalze mit einer pharmazeutisch annehmbaren Säure oder Base.

2. Verbindungen der Formel (I) nach Anspruch 1, worin R¹ und R², die an zwei benachbarten Kohlenstoffatomen vorliegen, gemeinsam mit den sie tragenden Kohlenstoffatomen eine Phenylgruppe oder substituierte Phenylgruppe bilden, deren Enantiomere und Diastereoisomere sowie deren Additionssalze mit einer pharmazeutisch annehmbaren Säure oder Base.

3. Verbindungen der Formel (I) nach Anspruch 1, worin R¹ eine Gruppe OR⁴ bedeutet, deren Enantiomere und Diastereoisomere sowie deren Additionssalze mit einer pharmazeutisch annehmbaren Säure oder Base.

4. Verbindungen der Formel (I) nach Anspruch 1, worin R³ ein Wasserstoffatom bedeutet, deren Enantiomere und Diastereoisomere sowie deren Additionssalze mit einer pharmazeutisch annehmbaren Säure oder Base.

5. Verbindungen der Formel (I) nach Anspruch 1, worin R³ eine Arylgruppe bedeutet, deren Enantiomere und Diastereoisomere sowie deren Additionssalze mit einer pharmazeutisch annehmbaren Säure oder Base.

6. Verbindungen der Formel (I) nach Anspruch 1, worin A eine Gruppe der Formel NR⁵R⁶ darstellt, deren Enantiomere und Diastereoisomere sowie deren Additionssalze mit einer pharmazeutisch annehmbaren Säure oder Base.

7. Verbindungen der Formel (I) nach Anspruch 1, die Dihydrobenzochromene oder Dihydrochromene sind, deren Enantiomere und Diastereoisomere sowie deren Additionssalze mit einer pharmazeutisch annehmbaren Säure oder Base.

8. Verbindungen der Formel (I) nach Anspruch 1, nämlich N-(9-Methoxy-2,3-dihydro-1H-benzo[f]chromen-2-yl)-acetamid, seine Enantiomere und Diastereoisomere sowie deren Additionssalze mit einer pharmazeutisch annehmbaren Säure oder Base.

9. Verbindungen der Formel (I) nach Anspruch 1, nämlich N-[2-(6-Methoxy-3,4-dihydro-2*H*-chromenyl)-ethyl]-acetamid, seine Enantiomere und Diastereoisomere sowie deren Additionssalze mit einer pharmazeutisch annehmbaren Säure oder Base.

10. Verbindungen der Formel (I) nach Anspruch 1, nämlich N-[(6-Methoxy-2*H-*3-chromenyl)-methyl]-butanamid, seine Enantiomere und Diastereoisomere sowie deren Additionssalze mit einer pharmazeutisch annehmbaren Säure oder Base.

11. Verfahren zur Herstellung der Verbindungen der Formel (I) nach Anspruch 1, **dadurch gekennzeichnet, daß** man als Ausgangsprodukt eine Verbindung der Formel (II) verwendet: 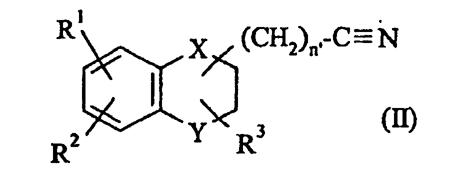 in der R¹, R², R³, X und Y die oben angegebenen Bedeutungen besitzen und n Werte von 0 bis 4 annehmen kann,
welche man:
◆ mit einem Reduktionsmittel behandelt zur Bildung der Verbindung der Formel (III): 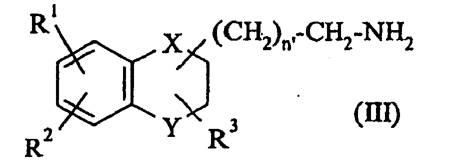 in der R¹, R², R³, X, Y und n' die oben angegebenen Bedeutungen besitzen, welche Verbindungen der Formel (III) weiterhin erhalten werden können
- durch Reduktion der Verbindung der Formel (IV): 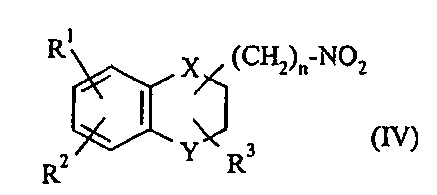 in der R¹, R², R³, X, Y und n die oben angegebenen Bedeutungen besitzen,
- oder ausgehend von der Verbindung der Formel (V): 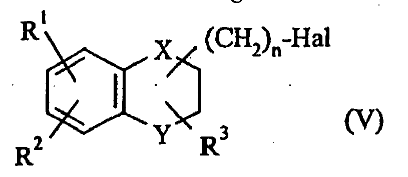 in der R¹, R², R³, X, Y und n die oben angegebenen Bedeutungen besitzen und Hal ein Halogenatom bedeutet,
die man mit einer Phthalimidgruppe substituiert und dann einer Hydrazinolyse unterwirft,
welche Verbindung der Formel (III) man mit:
- entweder einem Acylchlorid ClCOR⁸ oder dem entsprechenden (gemischten oder symmetrischen) Säureanhydrid, worin R⁸ die oben angegebenen Bedeutungen besitzt, kondensiert,
zur Bildung der Verbindung der Formel (I/a), einem Sonderfall der Verbindungen der Formel (I): 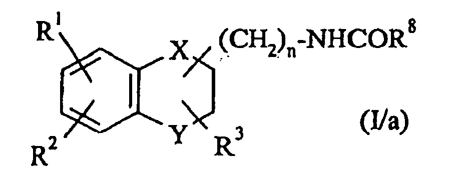 in der R¹, R², R³, R⁸, X, Y und n die oben angegebenen Bedeutungen besitzen,
welche man der Einwirkung eines Thionierungsmittels, wie dem Lawesson-Reagens unterwerfen kann zur Bildung der Verbindung der Formel (I/b), einem Sonderfall der Verbindungen der Formel (I): 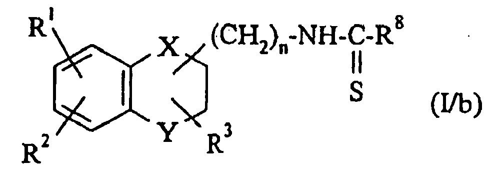 in der R¹, R², R³, R⁸, X, Y und n die oben angegebenen Bedeutungen besitzen,
- oder mit einer Verbindung der Formel (VI):
Z = C = N - R⁸ (VI)
in der Z und R⁸ die oben angegebenen Bedeutungen besitzen, kondensiert zur Bildung der Verbindung der Formel (I/c), einem Sonderfall der Verbindungen der Formel (I): 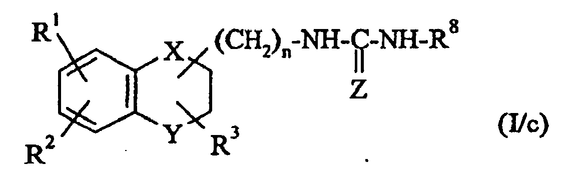 in der R¹, R², R³, R⁸, X, Y, Z und n die oben angegebenen Bedeutungen besitzen,
wobei die Gesamtheit der Verbindungen der Formeln (I/a), (I/b) und (I/c) die Verbindung der Formel (I/d) bilden, einem Sonderfall der Verbindungen der Formel (I): 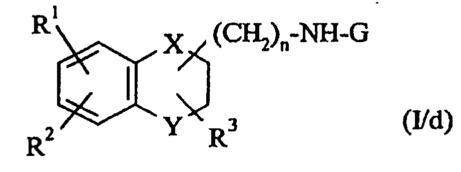 in der R¹, R², R³, X, Y und n die oben angegebenen Bedeutungen besitzen und G eine Gruppe COR⁸, CSR⁸ oder CZNHR⁸ darstellt, worin Z und R⁸ die oben angegebenen Bedeutungen besitzen,
welche man mit Hilfe einer klassischen Alkylierungsmethode unter Verwendung einer Verbindung der Formel (VII):
Alk - W (VII)
in der Alk eine geradkettige oder verzweigte (C₁-C₆)-Alkylgruppe und W eine austretende Gruppe, wie ein Halogenatom oder eine Tosylgruppe, bedeuten, oder mit Hilfe eines Dialkylsulfats alkylieren kann,
zur Bildung der Verbindung der Formel (I/e), einem Sonderfall der Verbindungen der Formel (I): 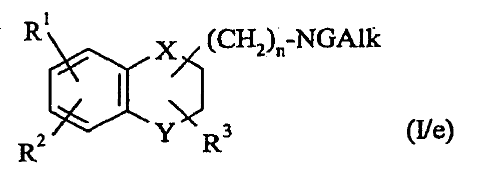 in der R¹, R², R³, X, Y, G, Alk und n die oben angegebenen Bedeutungen besitzen,
◆ oder in saurem oder basischem Medium hydrolysiert zur Bildung der Verbindung der Formel (VIII): 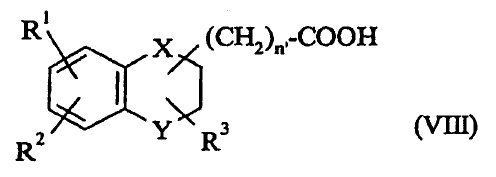 in der R¹, R², R³, X, Y und n' die oben angegebenen Bedeutungen besitzen, welche man nach der Aktivierung in Form des Säurechlorids oder in Gegenwart eines Kupplungsmittels der Einwirkung eines Amins H₂NR⁸ unterwirft, worin R⁸ die oben angegebenen Bedeutungen besitzt, zur Bildung der Verbindung der Formel (I/f), einem Sonderfall der Verbindungen der Formel (I): 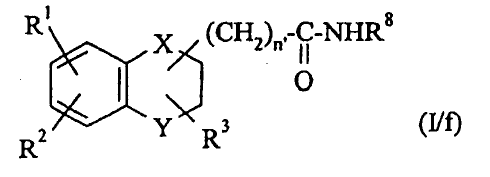 in der R¹, R², R³, R⁸, X, Y und n' die oben angegebenen Bedeutungen besitzen,
welche der Einwirkung eines Thionierungsmittels, wie des Lawesson-Reagens, unterworfen werden kann zur Bildung der Verbindung der Formel (I/g), einem Sonderfall der Verbindungen der Formel (I): 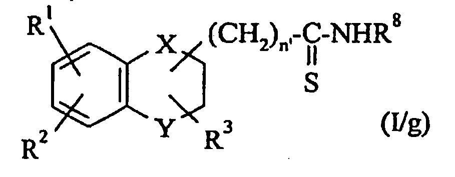 in der R¹, R², R³, R⁸, X, Y und n' die oben angegebenen Bedeutungen besitzen, welche Gesamtheit der Verbindungen (I/f) und (I/g), welche die Verbindung der Formel (I / h) bildet; 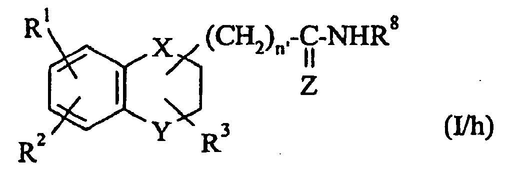 in der R¹, R², R³, R⁸, X, Y, Z und n' die oben angegebenen Bedeutungen besitzen,
mit Hilfe einer klassischen Alkylierungstechnik alkyliert werden kann zur Bildung der Verbindung der Formel (I/i): 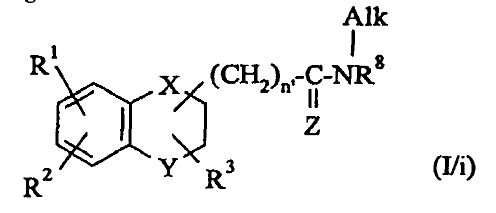 in der R¹, R², R³, R⁸, X, Y, Z, Alk und n' die oben angegebenen Bedeutungen besitzen,
welche Verbindungen der Formeln (I/a) bis (I/i), welche die Gesamtheit der Verbindungen der Formel (I) bilden, mit Hilfe einer klassischen Trennmethode gereinigt werden können, welche man gewünschtenfalls in ihre Additionssalze mit einer pharmazeutisch annehmbaren Säure oder Base überführen kann und welche man gegebenenfalls mit Hilfe einer klassischen Trennmethode in ihre Isomeren auftrennen kann.

12. Pharmazeutische Zubereitungen enthaltend als Wirkstoff mindestens eine Verbindung der Formel (I) nach einem der Ansprüche 1 bis 10 oder eines ihrer Additionssalze mit einer pharmazeutisch annehmbaren Säure oder Base in Kombination mit einem oder mehreren pharmazeutisch annehmbaren Hilfsstoffen.

13. Pharmazeutische Zubereitungen nach Anspruch 12, nützlich zur Behandlung von Störungen des melatoninergischen Systems.
